# EUROPEAN PATENT SPECIFICATION

(11) **EP 1 320 392 B1**
(45) Date of publication and mention of the grant of the patent: **05.03.2008**
(21) Application number: 01963359.3
(22) Date of filing: 30.08.2001
(51) Int. Cl.: A61M 1/00, A61N 1/00, A61B 5/04, A61N 1/05

(54) **INTRAVAGINAL DEVICE FOR ELECTRICALLY STIMULATING AND/OR FOR SENSING ELECTRICAL ACTIVITY OF MUSCLES AND/OR NERVES DEFINING AND SURROUNDING THE INTRAVAGINAL CAVITY**
INTRAVAGINALE VORRICHTUNG FÜR DIE ELEKTROSTIMULATION UND/ODER FÜR DIE MESSUNG ELEKTRISCHER AKTIVITÄT VON MUSKELN UND/ODER NERVEN, DIE DEN INTRAVAGINALRAUM DEFINIEREN ODER UMGEBEN
DISPOSITIF VAGINAL PERMETTANT DE STIMULER ELECTRIQUEMENT DES MUSCLES ET/OU DES NERFS DELIMITANT LA CAVITE VAGINALE ET L'ENVELOPPANT ET/OU D'EN DETECTER L'ACTIVITE ELECTRIQUE

(30) Priority: 31.08.2000 US 653263
(43) Date of publication of application: 25.06.2003
(73) Proprietor: Flexiprobe LTD., 52656 Ramat Gan (IL)
(72) Inventor: EINI, Meir, 74104 Nes Ziona (IL); TAMARKIN, Dov, 71908 Maccabim (IL); SARIG, Judith, 44235 Kfar Saba (IL); BAUR, Alfons Johannes, 19238 Doar Na Hevel Megiddo (IL)
(74) Representative: Schmitz, Jean-Marie
(86) International application number: PCT/IL2001/000824
(87) International publication number: WO 2002/017987

(56) References cited:
- US-A- 4 515 167
- US-A- 5 010 895
- US-A- 5 396 887
- US-A- 5 800 501
- US-A- 6 151 527

## Description

### FIELD AND BACKGROUND OF THE INVENTION

The present invention relates to an intravaginal device for electrically stimulating muscles and nerves defining and surrounding the intravaginal cavity and/or for sensing electrical activity of said muscles, and, more particularly, to a device and system utilizing same which are useful in preventing or treating pelvic floor dysfunction in women.

### Urinary incontinence

Urinary incontinence is characterized by the involuntary loss of urine in individuals. Urinary incontinence is typically brought on by sphincter dysfunction, and/or by weakening of the pelvic floor support, which weakening leads to a "dropped" bladder neck (a condition often termed as "Urethrocele"), or by bladder disorders.

Urinary incontinence affects approximately 13 million people in the United States alone, 85 % of them women. Urinary incontinence can be caused by mechanical stress (stress incontinence) typically brought on by heavy object lifting, coughing, laughing or sneezing, an uninhibited urge to void (typically referred to as urge incontinence) or by an uncontrollable slow leak (which is termed over flow incontinence), which is often experienced when incomplete bladder emptying is present. Additional but less common types of urinary incontinence include functional incontinence and unconscious or reflex incontinence.

Of the above causes for urinary incontinence, stress incontinence and urge incontinence are considered the most prevalent. In such cases, urine loss occurs when the intravesicle pressure (i.e., pressure within the urinary bladder) exceeds, even by a small amount of pressure, the maximal urethral closure pressure (i.e., pressure in the urethra to maintain closure). While the problem of stress incontinence occurs in both men and women, it predominantly occurs in women of childbearing age and beyond; the frequency of incontinence in women is approximately four times that of men. Less than one-third of women with moderate to severe incontinence are treated for the problem. While 25 to 41 % of all women suffer some form of incontinence, 6 to 8 % are troubled by the problem to the extent that they must wear diapers or sanitary napkins constantly.

There are several exercise programs, devices, and surgical procedures which can be used to alleviate urinary incontinence.

The "Kegel" exercise program is among the most basic and simple non- surgical alternative for treating urinary incontinence. Such an exercise program helps to strengthen the pelvic floor muscles to thereby treat urinary incontinence. Unfortunately, many women do not perform the "Kegel" exercises correctly, and as such, in most cases, significant improvement or alleviation is not achieved. In addition, recent studies have shown that the "Kegel" exercise program is only effective in cases of mild to moderate urinary incontinence.

Other non-surgical alternatives include diapers, which simply absorb the urine involuntarily voided, and as such do not alleviate the problem of incontinence. Such diapers also suffer from hygienic and aesthetic drawbacks; leakage occurs frequently, and there is no control over the urine loss.

A number of devices and plugs which are designed at preventing incontinence and which overcome such limitations, associated with diapers, have also been described in the prior art.

U.S. Pat. No. 4,457,299 to Corewell teaches an internally prestressed capsule device which is inserted into the urethra in order to aid in urinary incontinence.

U.S. Pat. No. 5,090,424 to Simon et al. describes a flexible urethral plug, designed for blocking involuntary urine flow through the urethra and assisting the natural function of the sphincter in closing the urethra.

Although such devices are functional in preventing accidental voiding of urine, they cannot be utilized to treat and/or alleviate the causes of urinary incontinence.

As such, a number of devices for treating urinary incontinence, which devices function in electrically stimulating the patient's musculature and/or monitoring muscle feedback have been described.

Electrical-stimulation (ES) has been found to be effective in increasing muscle strength while biofeedback monitoring of muscular activity is valuable in assessing muscle activity and thus promoting correct pelvic floor muscle control by the patient.

U.S. Pat. No. 4,396,019 to Perry, Jr., teaches the use of an electrode-carrying insert which functions in providing the patient with feedback on muscle activity and as such enables the patient to exercise self control over the musculature contributing to urinary incontinence.

U.S. Pat. NO. 4,881,526 to Johnson teaches of an intravaginal electrode and controller for preventing female urinary incontinence. The electrode includes an elongated and generally cylindrical carrier having a rounded tip, an extended lip, and a neck of reduced diameter. Motor receptor electrical stimulation signals received from the controller are coupled to the motor electrodes and directly stimulate pelvic floor musculature.

The rigid, non-yielding structure, of the above-described electrode carrying devices presents several disadvantages. Since contact between a wall of the intrabody cavity and an electrode of such devices is of utmost importance for efficient muscle activation, such devices must be fabricated in a variety of sizes to fit a variety of anatomical builds. In addition, the rigid construction of such devices interferes with the physiological movement of an exercising vaginal muscle. Furthermore, the rigidity of the device greatly increases patient discomfort.

To overcome these limitations, U.S. Pat No. 5,662,699 to Hamedi, teaches of a device which includes a flexible airtight sheath with a resilient skeleton and outer conductive bands which is collapsed by vacuum and inserted into the body cavity. When inflated within the cavity, the skeleton expands the sheath and forces the conductive bands against the body cavity wall thus ensuring optimal contact. Document US-A-5010895 discloses an expandable vaginal electrode.

This device is limited to recording muscle activity, no description is provided for its ability to induce muscle stimulation. In addition, although the use of such configuration overcomes the limitations inherent to rigid electrode carrying devices, the need for an inflating/deflating mechanism greatly complicates the fabrication and application of such a device and if air leakage should occur, also its reliability in interpretation of the results.

There is thus a widely recognized need for, and it would be highly advantageous to have, an intravaginal device capable of electrically stimulating, and/or recording the activity of, the musculature/nervure defining and surrounding the intravaginal cavity and yet devoid of the above mentioned limitations of prior art designs.

### SUMMARY OF THE INVENTION

The invention is as disclosed in the appended set of claims.

According to one aspect of the present invention there is provided a device for interacting with muscles and nerves defining and surrounding an intravaginal cavity of an individual, the device comprising (a) a body having memory properties such that when the body is contracted and positioned within an intravaginal cavity of the individual the body self expands to conform to a contour of the intravaginal cavity; and (b) at least one pair of electrodes being attached to an exterior surface of the body, such that when the body is positioned within the intravaginal cavity, each electrode of the at least one pair of electrodes is biased against a wall of the intravaginal cavity to thereby maintain electrical contact with the wall. The electrical current providable from the at least one pair of electrodes serves for electrically stimulating muscles and nerves defining and surrounding the intravaginal cavity.

According to further features in preferred embodiments of the invention described below, the device further comprising a power and control unit being for providing the electrical current to the at least one pair of electrodes, the power and control unit being attachable to connectors being in electrical communication with the at least one pair of electrodes.

According to still further features in the described preferred embodiments the connectors are positioned on a region of the body such that when the body is positioned within the intravaginal cavity and attached to the power and control unit, the power and control unit is positioned outside of the intravaginal cavity.

According to still further features in the described preferred embodiments the device further comprising at least one sensor attached to the body, such that when the body is positioned within the intravaginal cavity, the at least one sensor is capable of sensing muscle activity of the muscles and nerves defining and surrounding the intravaginal cavity, so as to serve for a loop feedback or biofeedback.

According to still further features in the described preferred embodiments the at least one sensor attached to the body is a pressure sensor or an electrical activity sensor.

According to still further features in the described preferred embodiments the at least one sensor attached to the body further includes a transmitter and an internal power source.

According to still further features in the described preferred embodiments the transmitter serves for transmitting a signal receivable outside the body, the signal including data pertaining to the muscle activity sensed by the at least one sensor.

The sensor may be a pressure sensor and may be connected to a biofeedback device for providing user sensible data to a user.

According to another aspect of the present invention there is provided a system for stimulating muscles and nerves defining and surrounding an intravaginal cavity of an individual, the system comprising: (a) an intravaginal positionable device assembly including: (i) a body having memory properties such that when the body is contracted and positioned within the intravaginal cavity of the individual the body self expands to conform to a contour of the intravaginal cavity; and (ii) at least one pair of electrodes being attached to an exterior surface of the body such that when the body is positioned within the intravaginal cavity, each electrode of the at least one pair of electrodes is biased against a wall of the intravaginal cavity to thereby maintain electrical contact with the intravaginal cavity wall, the electrical current providable from the at least one pair of electrodes serves for electrically stimulating muscles and nerves defining and surrounding the intravaginal cavity; and (iii) a power and control unit being detachably attached to the body and being for providing electrical current to the at least one pair of electrodes; and (iv) at least one sensor attached to the body, such that when the body is positioned within the intravaginal cavity, the at least one sensor is capable of sensing muscle activity of the muscles and nerves defining and surrounding the intravaginal cavity; and (v) a transmitter being for transmitting a signal receivable outside the body, the signal including data pertaining to the muscle activity sensed by the at least one sensor; and (b) an extra-corporeal monitoring unit being for processing the signal received from the transmitter to thereby determine duration or intensity of the electrical current provided to the at least one pair of electrodes from the power and control unit.

According to still further features in the described preferred embodiments the at least one pair of electrodes includes a plurality of electrode pairs, each pair of electrodes of the plurality of electrode pairs being attached to a specific region of the exterior surface of the body, such that each pair of electrodes is biased against a specific region of the wall of the intravaginal cavity when the body is positioned within the intravaginal cavity.

According to yet another aspect of the present invention there is provided a method of stimulating muscles and nerves defining and surrounding an intravaginal cavity of an individual, the method comprising the steps of: (a) positioning an intravaginal device within the intravaginal cavity of the individual, the intravaginal device including: (i) a body having memory properties such that when the body is contracted and positioned within an intravaginal cavity of the individual the body self expands to conform to a contour of the intravaginal cavity; and (ii) at least one pair of electrodes being attached to an exterior surface of the body such that when the body is positioned within the intravaginal cavity, each electrode of the at least one pair of electrodes is biased against a wall of the intravaginal cavity to thereby maintain electrical contact with the wall, wherein the electrical current providable from the at least one pair of electrodes serves for electrically stimulating muscles and nerves defining and surrounding the intravaginal cavity; and (b) providing the electrical current of a predetermined intensity and/or duration to the at least one pair of electrodes attached to the exterior surface of the body to thereby stimulate muscles and nerves defining and surrounding the intravaginal cavity of the individual.

According to still further features in the described preferred embodiments the system serves for treating or preventing a pelvic floor dysfunction such as, for example, urinary incontinence, in the individual.

According to still further features in the described preferred embodiments stimulation of the muscles and nerves defining and surrounding the intravaginal cavity serves for treatment or prevention of a female sexual arousal disorder.

According to still further features in the described preferred embodiments the body is constructed of an elastic material selected from the group consisting of silicon, rubber and latex. Other flexible materials are also envisaged.

According to still further features in the described preferred embodiments a width of the body when relaxed is at least 1-3-1.5 times larger than the width of the body when fully contracted.

According to still further features in the described preferred embodiments a length of the body when relaxed is 1.3-1.7, preferable 1.5, times shorter than the length of the body when fully contracted.

According to still further features in the described preferred embodiments the at least one pair of electrodes include a conductive material selected from the group consisting of gold, carbon, silver, stainless steel platinum and/or any metal alloy or composite capable of electrical conductance.

According to still further features in the described preferred embodiments the at least one sensor attached to the body is a pressure sensor or an electrical activity sensor.

According to still further features in the described preferred embodiments the power and control unit includes a receiver for receiving command signal from the extracorporeal unit, the command signals serving for modulating the duration or the intensity of the electrical current provided to the at least one pair of electrodes from the power and control unit.

According to still further features in the described preferred embodiments the body defines a substantially rounded diamond shaped frame when relaxed.

According to still further features in the described preferred embodiments stimulation of the muscles and nerves defining and surrounding the intravaginal cavity serves for treatment or prevention of a pelvic floor dysfunction such as, for example, urinary incontinence, in the individual.

According to still further features in the described preferred embodiments the device includes a plurality of electrode pairs, each pair of electrodes of the plurality of electrode pairs being positioned at a specific region of the exterior surface of the body, whereas each pair of the plurality of electrode pairs is independently provided with an electrical current of a specific frequency, intensity and/or duration.

According to another aspect of the present invention there is provided a device for sensing electrical activity of muscles defining and surrounding an intravaginal cavity of an individual, the device comprising (a) a body having memory properties such that when the body is contracted and positioned within an intravaginal cavity of the individual the body self expands to conform to a contour of the intravaginal cavity; and (b) at least one pair of electrodes being attached to an exterior surface of the body such that when the body is positioned within the intravaginal cavity, each electrode of the at least one pair of electrodes is biased against a wall of the intravaginal cavity to thereby maintain electrical contact with the wall.

The present invention successfully addresses the shortcomings of the presently known configurations by providing an intravaginal device for stimulating the muscles and nerves defining and surrounding the intravaginal cavity and/or for sensing electrical activity of said muscles, which device can be fitted to a wide range of anatomical builds, is easy to position while remaining at the desired location and orientation within the intravaginal cavity, and yet minimizes patient discomfort when utilized.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a perspective view of a device for stimulating muscles and nerves defining or surrounding the intravaginal cavity and/or sensing activity of said muscles, according to the teachings of the present invention;
FIG. 2 a perspective view of a power and control unit attachable to the device described in Figure 1;
FIG. 3 is a side view of the device shown in Figure 1 connected to the power and control unit shown in Figure 2;
FIGs. 4a-b demonstrate the process of inserting and positioning the device for stimulating muscles and nerves defining or surrounding the intravaginal cavity, and/or sensing activity of said muscles, according to the teachings of the present invention;
FIG. 5 shows a system in accordance with the teachings of the present invention;
FIGs. 6 - 15 show varying levels of detail of embodiments of an intravaginal device according to the present invention,

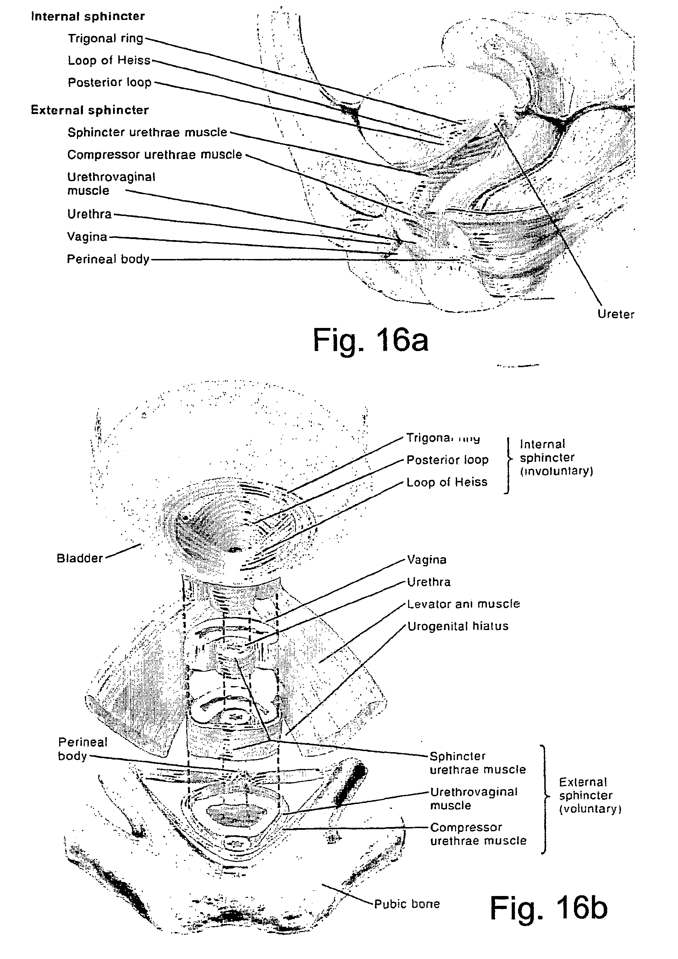

FIGs. 16a-b illustrate the anatomical positioning of the internal and external sphincters (reproduced from "Clinical Symposia Vol. 47, 3 Urinary Incontinence in Women" Ciba Pharmaceutical Div. Ciba-Geigy Corp. Summit, New Jersey);

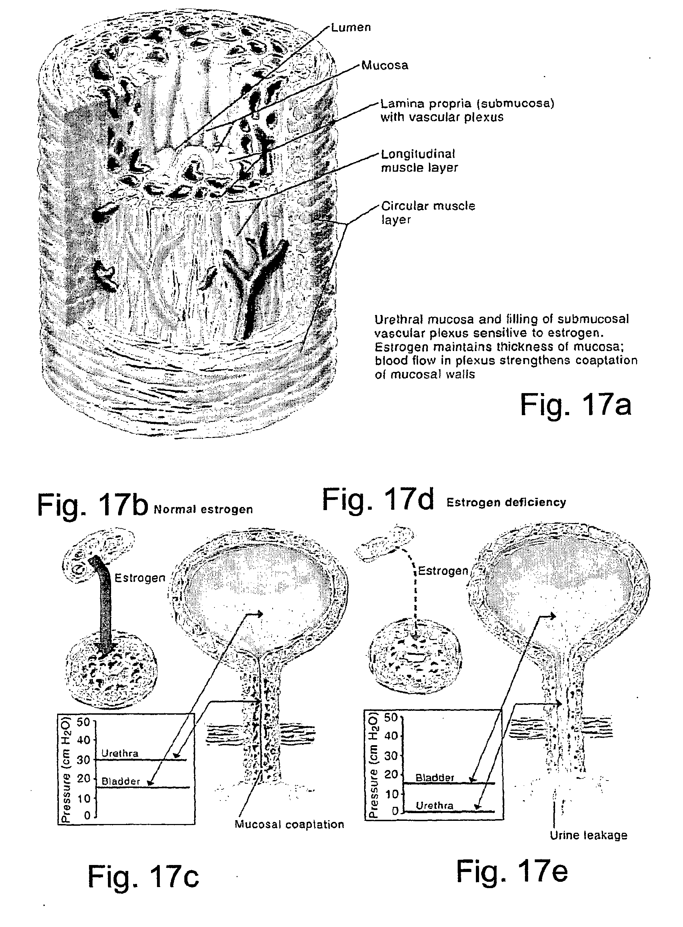

FIGs. 17a-e illustrate urethral mucosal coaptation (reproduced from "Clinical Symposia Vol. 47, 3 Urinary Incontinence in Women" Ciba Pharmaceutical Div. Ciba-Geigy Corp. Summit, New Jersey);
FIGs. 18a-b illustrate pubocervical fascia support of the bladder, urethra and vagina (reproduced from "Clinical Symposia Vol. 47, 3 Urinary Incontinence in Women" Ciba Pharmaceutical Div. Ciba-Geigy Corp. Summit, New Jersey);
FIGs. 19a-e illustrate the anatomical structures and physiological factors underlying stress incontinence in women (reproduced from "Clinical Symposia Vol. 47, 3 Urinary Incontinence in Women" Ciba Pharmaceutical Div. Ciba-Geigy Corp. Summit, New Jersey);
FIGs. 20a-g illustrate the physiological factors underlying detursor instability and hyperreflexia (reproduced from "Clinical Symposia Vol. 47, 3 Urinary Incontinence in Women" Ciba Pharmaceutical Div. Ciba-Geigy Corp. Summit, New Jersey);
FIGs. 21a-h illustrate various causes of incontinence associated with anatomical abnormalities caused by bladder neck suspension surgery (Figures 11b-e) and various other factors (Figures 11f-g) (reproduced from "Clinical Symposia Vol: 47, 3 Urinary Incontinence in Women" Ciba Pharmaceutical Div. Ciba-Geigy Corp. Summit, New Jersey); and
FIGs. 22a-c illustrate the physiological factors and anatomical structures participating in the filling and emptying phases of bladder function (reproduced from "Clinical Symposia Vol. 47, 3 Urinary Incontinence in Women" Ciba Pharmaceutical Div. Ciba-Geigy Corp. Summit, New Jersey).

### DESCRIPTION OF THE PREFERRED EMBODIMENTS

The present embodiments comprise an insertion device, for example an intravaginal device, which can be used to electrically stimulate the muscles and nerves defining and surrounding the intravaginal cavity and/or sense, record and report the muscles electrical or mechanical activity. Specifically, the present embodiments can be used to treat female pelvic floor dysfunction leading to, for example, urinary incontinence, by electrically stimulating the muscles responsible for such a condition and/or by monitoring the activity of such muscles so as to provide information to a biofeedback system.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

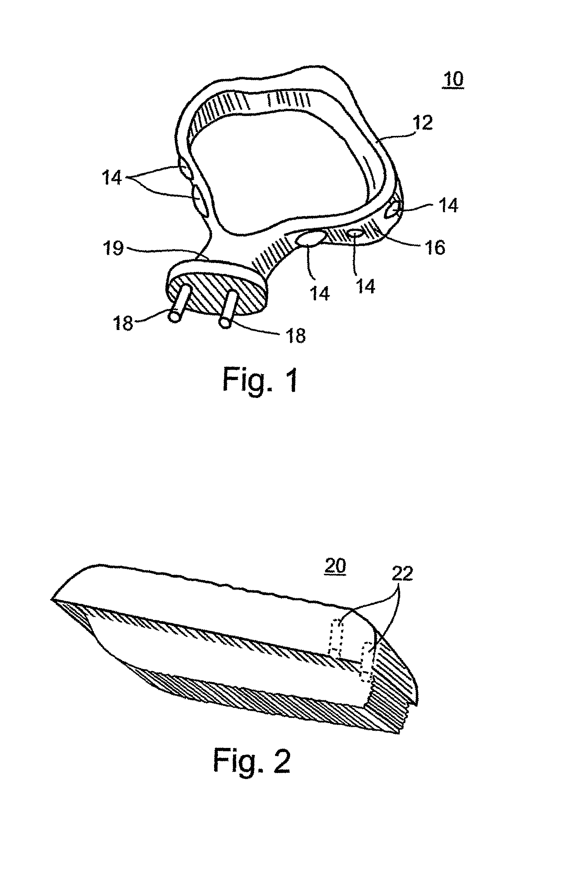

Referring now to the drawings, Figure 1 illustrates one possible configuration of a device for stimulating, and/or sensing electrical activity of, muscles and nerves defining and surrounding an intravaginal cavity of an individual, which is referred to hereinunder as device **10.**

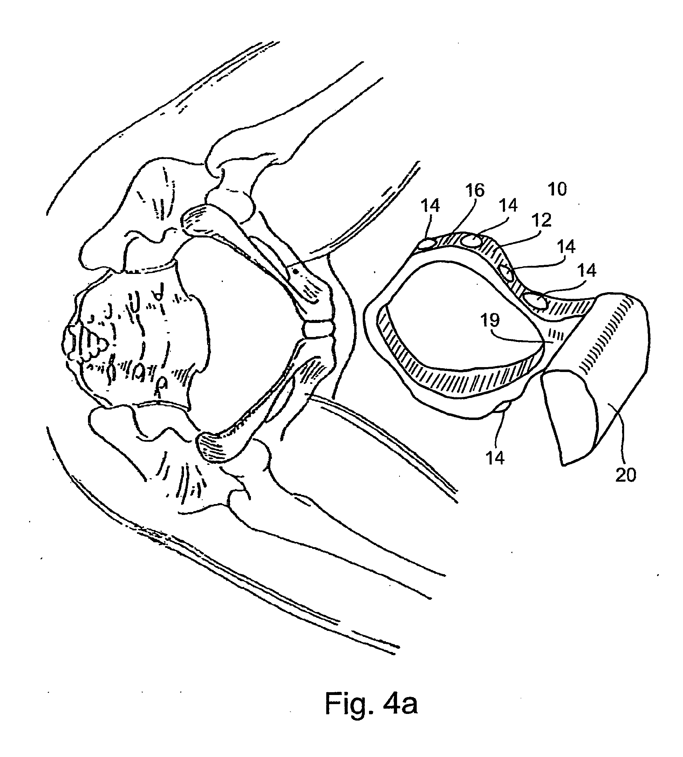

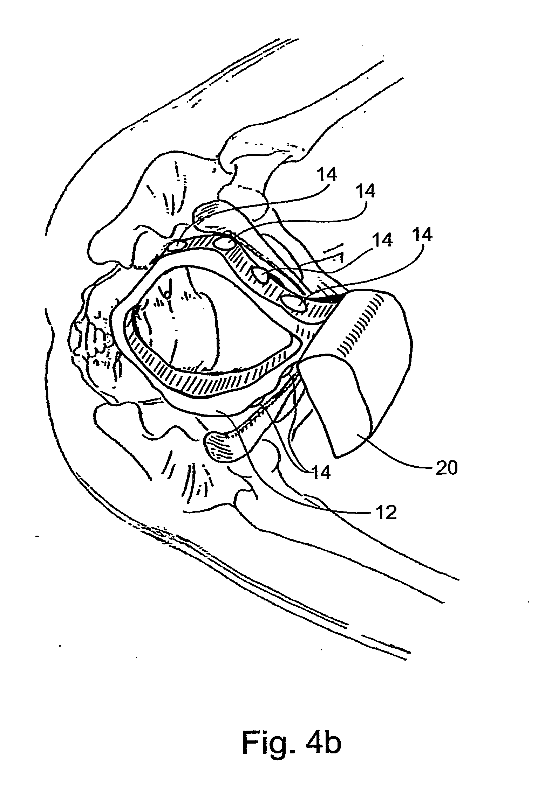

Device **10** includes a body **12.** Body **12** is constructed such that when contracted and positioned within an intravaginal cavity of the individual (see Figures 4a-b), body **12** self expands to conform to a contour of the intravaginal cavity. This memory (self expansion) property of body **12** can be achieved by fabricating at least a portion of body **12** from an elastic material having such memory, such as, but not limited to, silicon, rubber, latex, or alternatively, by providing various sprigged hinge points along body **12** which allow contracting of body **12** against a force of a spring, thus allowing body **12** to self expand following contraction.

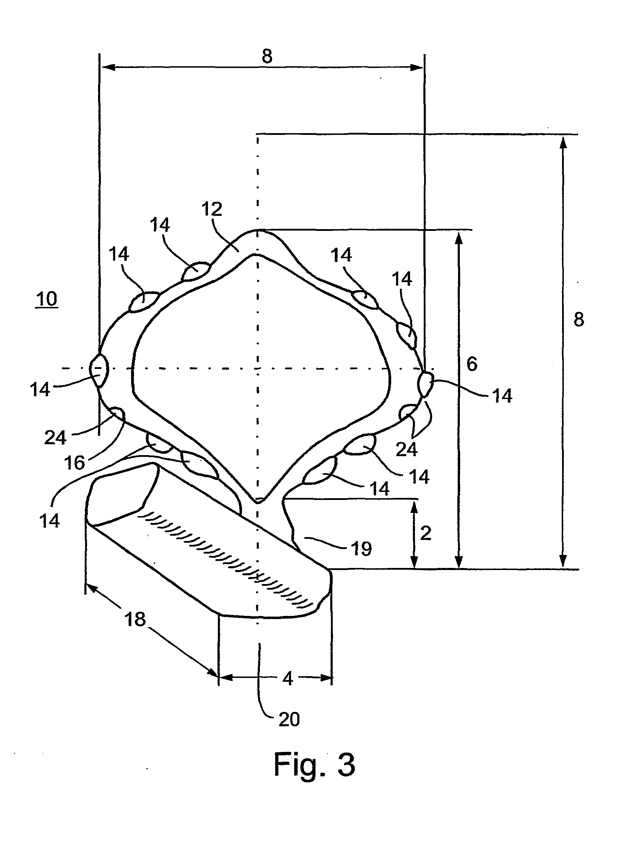

As best seen in Figure 3, device **10** further includes at least one pair of electrodes **14.** In one embodiment of the present invention some or all of electrodes **14** serve for providing an electrical current to the walls of the intravaginal cavity of the individual. In another embodiment of the present invention some or all of electrodes **14** serve for recording electrical activity of the muscles defining the walls of the intravaginal cavity of the individual. In either case, electrodes **14** are preferably attached to an exterior surface **16** of body **12.** Thus, when body **12** is positioned within the intravaginal cavity, each electrode **14** is biased against a wall of the intravaginal cavity to thereby maintain electrical contact with the wall.

Preferably, device **10** includes several pairs of electrodes **14,** each pair being attached to a specific region of exterior surface **16.** According to this preferred configuration of device **10,** each pair of electrodes is biased against (contacts) a specific region of the intravaginal cavity wall when device **10** is positioned within the intravaginal cavity.

As is further detailed in the Examples section which follows, this configuration of device **10** of the present invention is particularly advantageous since it enables specific stimulation (electrical) of specific regions of the intravaginal cavity wall according to a predetermined stimulation pattern.

To maintain optimal electrical conductance, the portion of electrodes **14** which maintains contact with the intravaginal cavity wall is fabricated from a material such as, but not limited to, gold and platinum, or any other highly conductive material including metal alloys and composites.

It will be appreciated that when expanding to its relaxed state, body **12** causes reduction (anatomical repositioning) of prolapsed intravaginal tissue and therefore, to the benefit of more physiological muscular exercising. The flexibility of body **12** in itself, as opposed to rigid constructions, also adds to achieving more physiological muscular exercising.

The electrical current provided from electrodes **14** serves for electrically stimulating muscles and/or nerves defining and surrounding the intravaginal cavity.

Preferably, such muscles include, the pelvic floor muscles, which form a striated muscular sheet through which viscera pass. This striated muscle sheet is divided into two main layers. The deep layer (termed the Levator Ani) is actually subdivided into the Pubococcygeus (which further includes the Puborectalis), the Ileococcygeus, and the Ischiococcygeus. These muscles are defined by the area of attachment to the pubic bone, from which they run, along the arcus tendineus (a condensation of the obturator fascia), through the Ischial Spines to the Coccyx and Sacrum.

The superficial layer includes the muscles of the perineum which are positioned between the lower part of the pubic bone, the ischial tuberosities, and the coccyx. These muscles are attached to a central tendon, named the perennial body, which lies between the urogenital viscera and the anal canal. These muscles include the superficial and deep transverse perinei muscles, the ventral extension of the external sphincter muscle which forms a tendinous intersection with the Bulbocavernosus and Ischicavernosus muscles and the external anal sphincter, which is located in the posterior compartment of the Perineum.

The weakening or dysfunction of one or more of these muscles or the nerves controlling the function of such muscles contributes to the onset and progression of pelvic floor dysfunction, including urinary incontinence, fecal incontinence, voiding difficulties, prolapsed genital organs and sexual dysfunction (e.g., FSAD).

Thus, by stimulating such musculature and/or nerves, device **10** of the present invention can be utilized to prevent or treat pelvic floor dysfunction such as, for example, urinary incontinence. The Examples section below provides further description on various treatment regimens, which can be effected by device **10** of the present invention.

It will be appreciated that strengthening the pelvic floor muscles also improves the ability to experience stronger and/or longer orgasms when engaged in sexual activity.

Since the position of these muscles, in relation to the intravaginal cavity varies from one individual to the next, placement of electrodes **14** upon exterior surface **16** is of crucial importance when wanting to achieve maximal stimulation/activity records in each individual. It will further be appreciated that although optimal placement of electrodes **14** can be achieved by testing the device on a large number of individuals, such anatomical limitations can be traversed by placing several pairs of electrodes **14** upon exterior surface **16** as is mentioned hereinabove thus enabling the activation of one or more specific pairs of electrodes **14** according to individual anatomical built. This may also be advantageous in cases wherein muscles of one side of the intravaginal cavity are stronger then the muscles of the other side as is typically the case in birth related incontinence. Thus, muscle stimulation with the device of the present invention may be applied preferentially or solely to the weak muscles, as opposed to all of the muscles surrounding and defining the vaginal cavity.

As shown in Figures 1 and 3, one possible configuration of body **12** is a substantially rounded diamond shaped frame of approximately 5-7 cm in length and 7-9 cm in width in its relaxed state. In this configuration, electrodes **14** are preferably positioned on exterior surface **16** of the members defining such a frame.

The construction of body **12** is selected such that when relaxed it is 1.3-1.7, preferable 1.5, times shorter than when fully contracted. Similarly, the width of body **12** is selected such that when relaxed is at least 1.3-1.7, preferable 1.5, times larger than when fully contracted. This enables body **12** to be utilizable in a wide range of anatomical builds.

Device **10** further includes connectors **18,** which serve for electrically interfacing with a power and control unit, which is further described below.

Preferably, connectors **18** are positioned on a neck **19** of body **12** such that when body **12** is positioned within the intravaginal cavity and attached to the power and control unit, the power and control unit is positioned outside of the intravaginal cavity.

Thus, as is specifically shown in Figures 2-5, device **10** further includes a power and control unit **20,** which serves for providing electrical currents or to electrodes **14** and/or for creating a potential difference therebetween. Power and control unit **20** includes receptacles 22, which serve for interfacing with connectors **18** described above.

Power and control unit **20** preferably also includes an attachment mechanism for securing power and control unit **20** to the underwear or body of the individual when device **10** is in use. This ensures that body **12** does not translate or rotate inside the intravaginal cavity when in use, thus guaranteeing optimal electrical contact and stimulation of the desired muscles. Such attachment mechanism can include Velcro^{™} fasteners for securing power and control unit **20** to the underwear or pubic hair of the individual, or it can include sticky tape or suction cups for securing power and control unit **20** against the body of the individual.

Power and control unit includes a power source, such as a battery, a control unit for controlling the output from the power source and the required circuitry. Power and control unit **20** preferably also includes a processing unit as is further detailed hereinbelow.

Power and control unit **20** preferably also includes exterior controls for controlling the intensity, frequency and duration of the electrical current provided to electrodes **14.** In device **10** which includes more than one pair of electrodes **14,** such exterior controls can also be used to separately control each pair of electrodes **14,** such that an intensity, frequency and/or duration of an electrical current delivered from each pair of electrodes **14** can be independently controlled. In addition, power and control unit **20** can also be preprogrammed to deliver a preset pattern of stimulation via one or more pairs of electrodes **14.**

For example, an individual or a treating physician can set the power and control unit according to individual needs, or activate a preset program of stimulation in order to effectively stimulate and thus contract and exercise the musculature defining or surrounding the intravaginal cavity. The Examples section, which follows, details various stimulation regimens which are suitable for incontinence treatment and which can be provided by device **10** of the present invention

Preferably, electrical current is provided from power and control unit **20** in an intermittent pattern, which includes 1-20, preferably 2-10 seconds intervals each providing alternating or direct current spaced by 2-40, preferably 4-20 seconds of rest intervals. Generally a pulse width of 100 - 200 microseconds is used with intervals to give a frequency of 5 - 90Hz.

As shown in Figure 3, according to a preferred embodiment of the present invention device **10** further includes at least one sensor **24** (two are shown in Figure 3) which is preferably attached to exterior surface **16** of body **10.** Sensor(s) **24** serve for sensing muscle activity of the muscles defining and surrounding the intravaginal cavity. Such sensing can be either: prior to, during, or following stimulation of these muscles via electrodes **14.**

Sensor(s) can be either pressure sensors, or sensors which are capable of sensing muscle electrical activity (e.g., surface electromyography sensors). It will be appreciated that, in the latter case a grounding electrode must be included in device **10.**

In any case, sensors **24** serve to evaluate muscle activity prior to, during or following treatment to thereby serve as either a basis for a treatment regimen or as feedback to a treatment regimen.

According to one preferred embodiment of the present invention, sensors **24** interface with power and control unit **20** via connectors **18** or any other dedicated connectors preferably positioned at neck **19.** To this end, sensors **24** are provided with power from power and control unit **20** and also communicate information relating to muscle activity to power and control unit **20.**

Such information received by power and control unit 20 can be processed by a processing unit contained therein and the processed information utilized to automatically adjust or set the intensity and/or duration of the electrical current provided from power and control unit **20** to electrodes **14.**

It will be appreciated that power and control unit **20** can also include a memory device and ports for interfacing with a personal computer. Thus, sensor information of each treatment session, which is collected by power and control unit **20,** can be uploaded onto a computer for storage and/or further analysis.

The above-described configuration of device **10** of the present invention is particularly advantageous since it enables device **10** to set the most suitable course of treatment for an individual.

Alternatively, the information provided by the sensor can be transmitted to an extra-corporeal unit for analysis via a transmitter included within sensor(s) **24** or body **12** or preferably within power and control unit **20.**

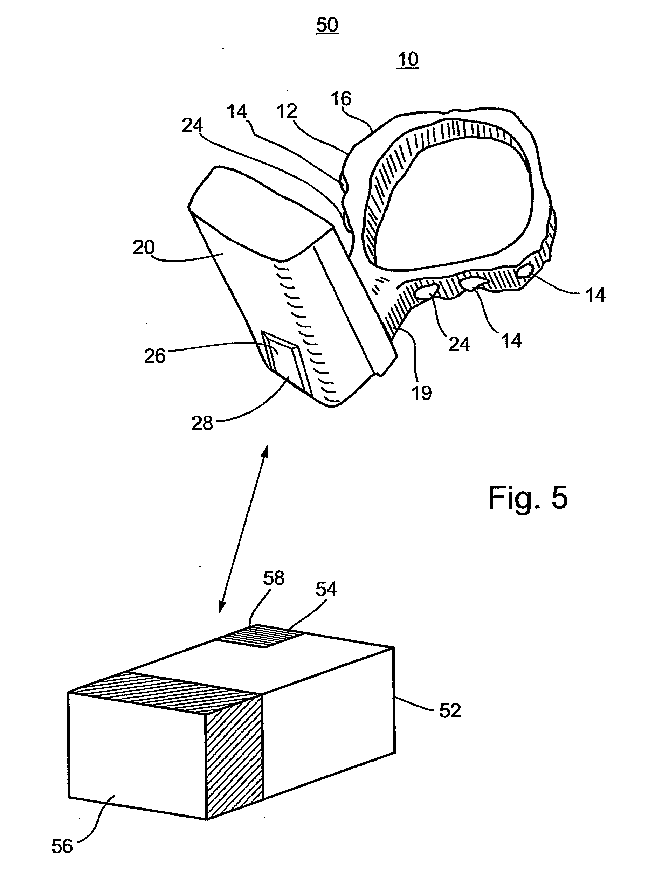

Thus, according to another aspect of the present invention and as shown in Figure 5, device **10** of the present invention can form a part of a system for stimulating muscles and nerves defining and surrounding an intravaginal cavity of an individual, which is referred to hereinunder as system **50.**

System **50** includes device **10** as described above, which according to this aspect of the present invention includes a transmitter **26** preferably positioned on or in power and control unit **20.** Transmitter **26** serves for transmitting a signal receivable outside the body, which signal includes data pertaining to muscle activity sensed by sensor(s) **24.**

System **50** also includes an extracorporeal unit **52** which serves for processing the signal received from transmitter **26** to thereby determine duration or intensity of the electrical current provided to electrodes **24** from power and control unit **20.** To this end, extracorporeal unit **52** includes a receiver **54** and a processing unit 56 (e.g., a personal computer).

Preferably, power and control unit **20** of device **10** also includes a receiver **28.** Thus, following processing of the signal, received from transmitter **26,** extracorporeal unit **52** preferably transmits, via a transmitter **58,** a command signal receivable by receiver **28,** which command signal determines the duration or intensity of the electrical current provided to electrodes **24** from power and control unit **20.**

It will be appreciated that the above-described configuration of system **50** is advantageous since extracorporeal unit **52** enables a more accurate processing of the information collected by sensor(s) **24** to thereby more accurately determine a suitable course of treatment. In addition, this configuration of system **50** also enables processing and storing of information collected from several treatment sessions conducted over any period of time of say weeks or months. Furthermore, statistical analysis of information collected from several individuals can be utilized to improve treatment regimens or to improve the construction of device **10.**

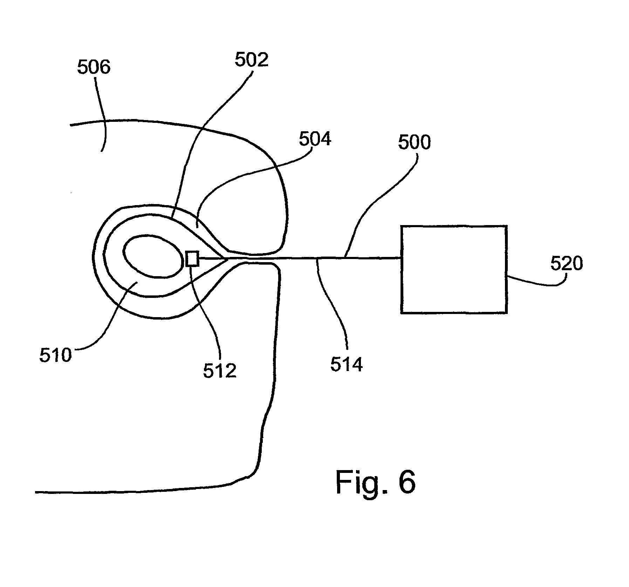

Reference is now made to Fig. 6, which is a therapeutic system 500 according to an embodiment of the present invention. System 500 is shown inserted in a cavity 504 of a body 506, and may be used for measuring for measuring forces acting to compress body cavity 504, such as forces generated by the contraction of muscles constituting or in proximity to cavity 504.

System 500 comprises a measurement device 502, preferably of annular shape. Alternatively, the shape of device 502 is a modified annular form characterized by a rounded diamond shape. Device 502 is flexible, and its shape is capable of distortion in response to pressures exerted thereupon.

Device 502 comprises a flexible container 510, preferably of silicon, and an electronic measuring device 512 at least partially contained therein. Measuring device 512 is operable to respond quantitatively to deformations in the shape of container 510.

Device 502 further comprises an output apparatus 514 for transmitting data from measuring device 512 to an data utilization system 520. Data utilization system 520 is preferably external to device 502.

Output apparatus 514 is any apparatus operable to transmit data between measuring device 512 and data utilization system 520. In a preferred embodiment output apparatus 514 is simply a wired connection between measuring device 512 and data utilization system 520. In alternative constructions, output apparatus 514 is a data transmission device such as a radio-frequency data transmission module, a fiber-optic connection, or an infra-red data transmission module.

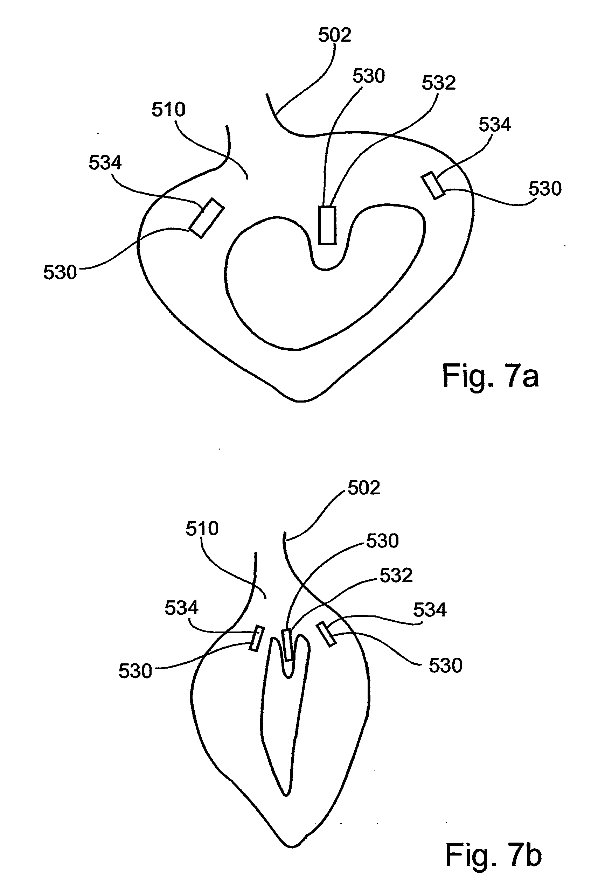

Reference is now made to figures 7a and 7b, which comprise a first alternative construction of device 502, according to a preferred embodiment of the present invention. In the embodiment of figure 7a, electronic measuring device 512 comprises a signal strength measurement sensor 530. Signal strength sensor 530 preferably comprises a signal generator 532 operable to generate a signal, and at least one signal receiver 534, operable to receive the signal generated by signal generator 532.

Signal receiver 534 is preferably mechanically flexibly connected to signal generator 532. In a preferred embodiment, flexible connection is provided by flexible container 510, which is constructed of a semi-rigid yet flexible material. The shape of container 510 is subject to change and distortion in response to pressures exerted on container 510 from external sources, yet the material of container 510 will tend to return to its shape when such external pressures are relaxed.

In consequence of the flexible connection between signal generator 532 and signal receiver 534, changes in external forces acting on container 510, and consequent changes in the shape of container 510 as a result of those forces, causes a change in the spatial relationship between signal generator 532 and signal receiver 534. The signals generated by signal generator 532 are of a strength such that changes to the distance, or other aspects of the spatial relationship, between generator 532 and receiver 534 affects the strength of the signal as received by receiver 534. Thus, for example, in a particular configuration, external forces compressing portions of container 512 might cause generator 532 to move closer to receiver 534, thereby increasing the strength of the received signal detected by receiver 534. Similarly, relaxation of external forces, allowing container 510 to return to its relaxed form, might then move generator 532 further from receiver 534, thereby weakening the signal received by receiver 534.

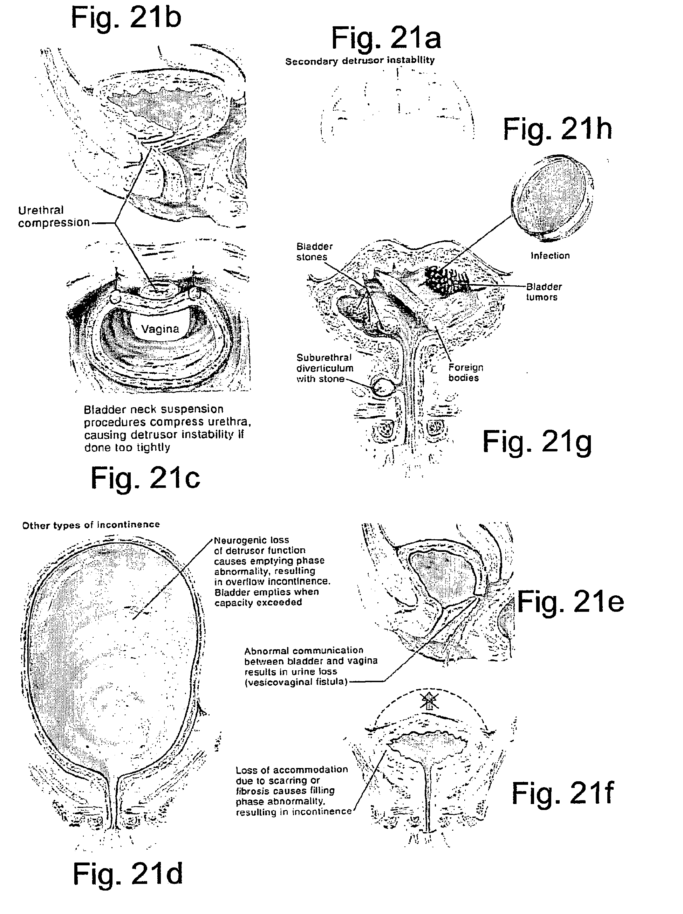

In figure 7a, device 502 is shown with container 510 in its relaxed state, with signal receivers 534 relatively distant from signal source 532. Figure 21b, by way of contrast, shows device 502 as it is when container 510 is compressed by external forces, causing signal receivers 534 to be moved closer to signal source 532.

In a preferred embodiment, generator 532 is a generator of electronic signals. Typically, signals generated by generator 532 may be pulse signals, or wave signals, or modulated pulse signals, or modulated wave signals, or changing pulse signals, or changing wave signals, and changing frequency signals or phase shifted signals.

In alternative preferred constructions, generator 532 is an infrared transmitter and signal receiver 534 is an infrared receiver.

In an additional alternative preferred construction, generator 532 is a light source and receiver 534 is a light-sensitive sensor.

In an additional alternative preferred construction, generator 532 is a permanent magnet generating a magnetic field, and signal receiver 534 is a magnetic sensor, such as a Hall effect semiconductor receiver.

In an additional alternative preferred construction, generator 532 is an electro-static field generator utilizing a plate antenna, and signal receiver 534 is an electro-static field sensor utilizing a plate antenna.

In an additional alternative preferred construction, generator 532 is an ultrasound sound generator, and receiver 534 is an ultrasound sound sensor. In a further preferred embodiment, generator 532 is a sound source such as a loudspeaker, and receiver 534 is a sound sensor such as a microphone.

In an additional alternative preferred construction, generator 532 is a very low radiation isotope, and signal receiver 534 is a radiation sensor operable to measure an amount of received radiation.

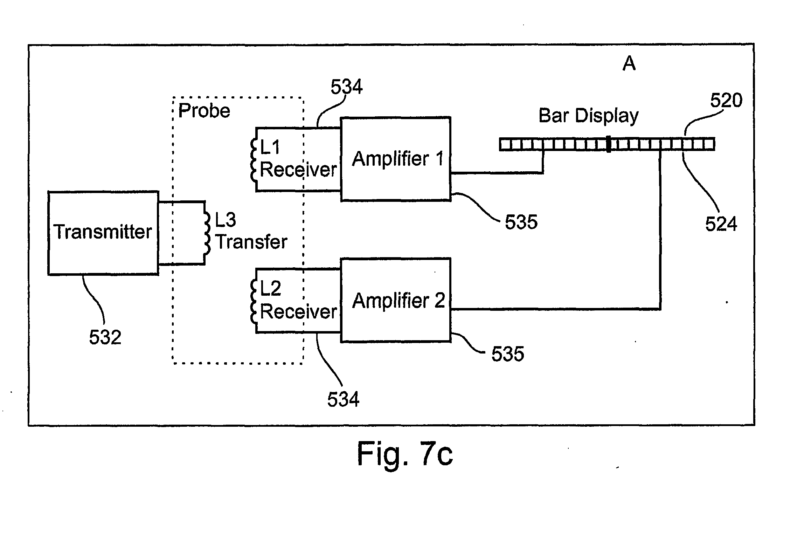

Attention is now drawn to Figure 7c, which is a block diagram presenting a simplified schematic of an embodiment according to the present invention, wherein is shown electrical connections consistent with the embodiment presented by figures 7a and 7b, and showing an additional amplification stage provided by signal amplifiers 535 for amplifying signals received by signal receiver 534, and further presenting external data utilization system 520, here implemented as a bar graph display 524.

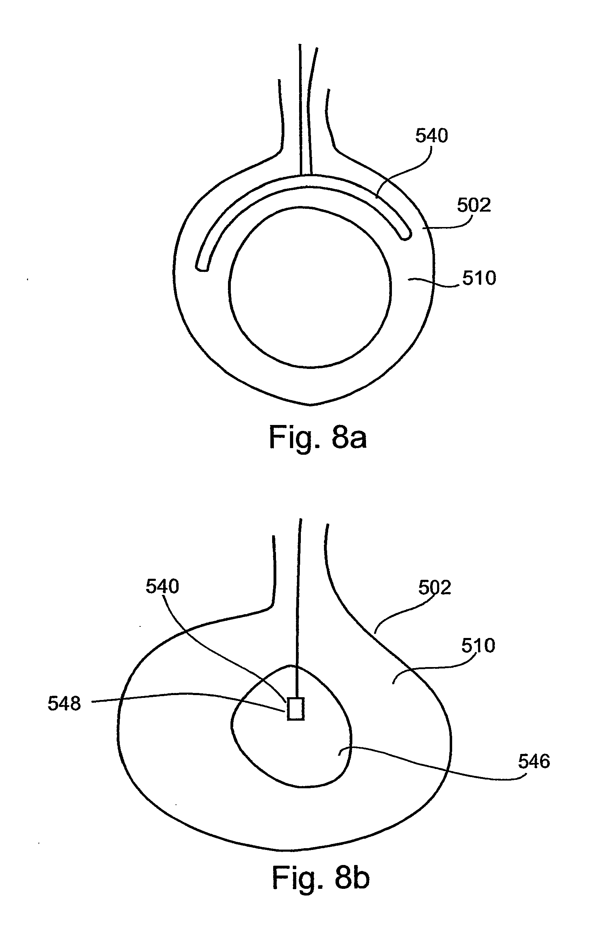

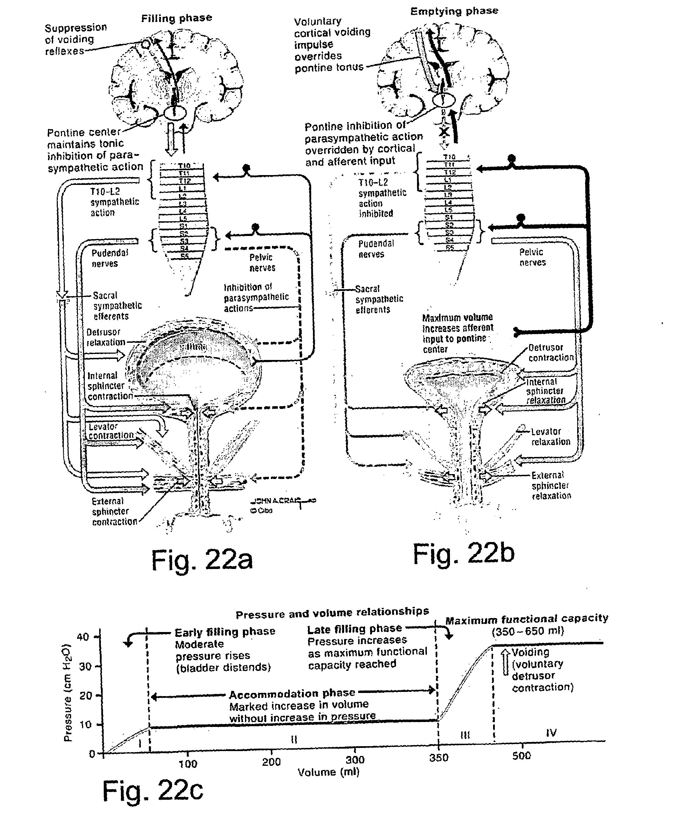

Attention is now drawn to Figures 8a and 8b, which present an additional alternative construction of a device according to a preferred embodiment of the present invention. A variable resistance element 540 presents an electrical resistance which varies as a function of stress or pressure applied to element 540. In the construction presented in Figure 22a, forces tending to compress container 510 will tend to bend variable resistance element 540 which is a stress sensitive resistor 544, thereby changing its resistance. A standard voltage is applied to variable resistance element 540, and voltage drop across variable resistance 540 is measured by a voltmeter, or current in the circuit measured by an ammeter, thereby obtaining a measure of the compression forces to which container 510 is being subjected or of the size to which container 510 has been compressed.

In an alternative construction presented in Figure 8b, container 510 entirely contains a gas-filled ballon-like partition 546, which also contains variable resistor 540 which is a pressure-sensitive resistor 548. Compression of container 510 by outside forces causes compression of the gas contained in partition 546. This change in pressure causes a change in electrical resistance of pressure-sensitive resistor 548. A standard voltage is applied to resistor 548, and voltage drop across resistor 548 is measured by a voltmeter, or current in the circuit is measured by an ammeter, thereby obtaining a measure of the compression forces to which container 510 is being subjected, and thereby, indirectly, a measure of the size to which container 510 has been compressed.

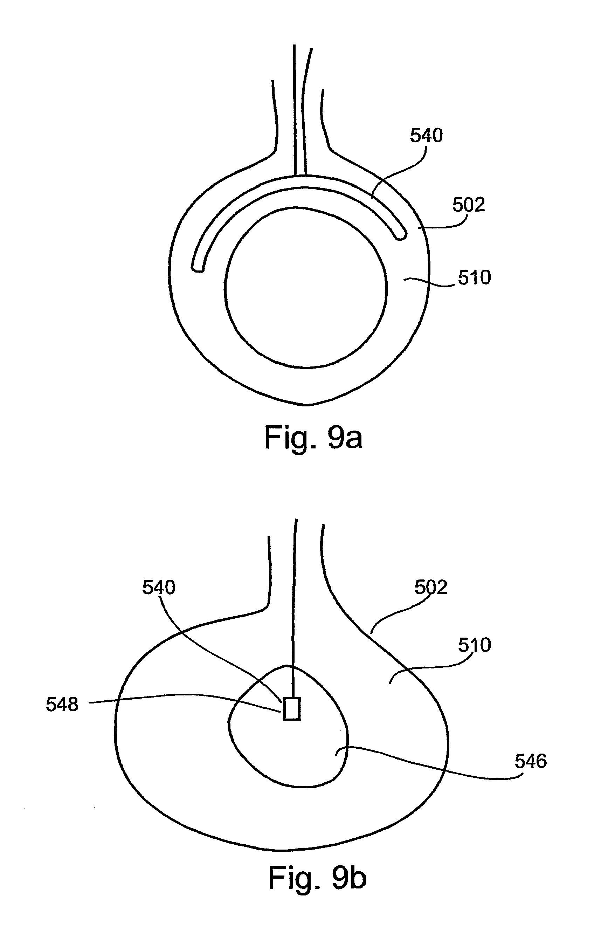

Attention is now drawn to Figures 9a and 9b, which present alternative constructions for a measuring device according to the present invention, wherein forces exerted on the device 502 are measured by changes in capacitance of a pressure-sensitive capacitance device.

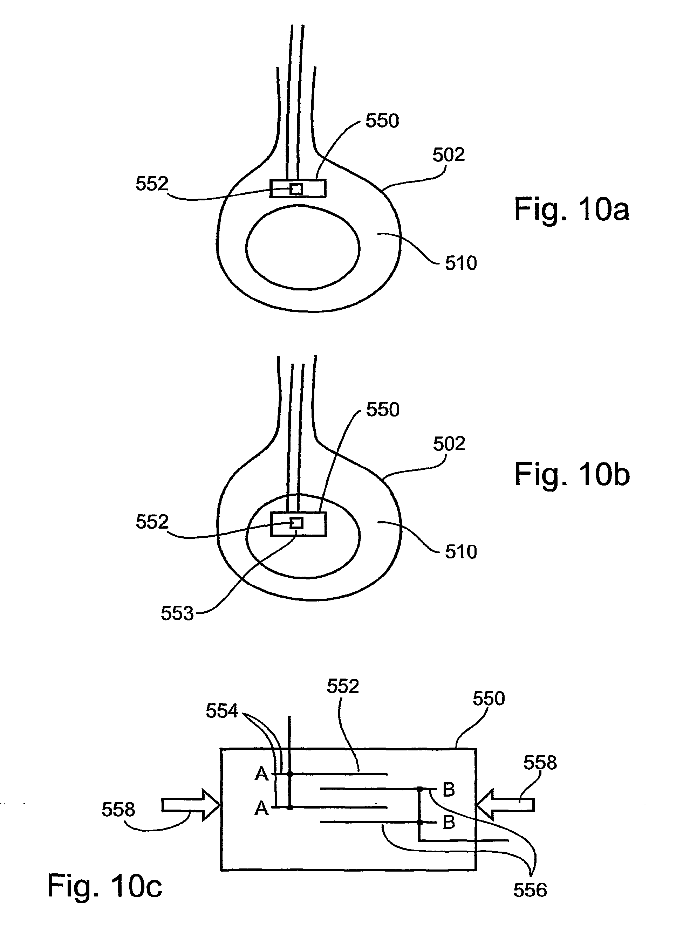

Figures 10a and 10b present measuring device 502 wherein electronic measuring module 512 comprises a capacitance measurement tool 550 operable to respond quantitatively to changes in capacitance of a capacitance module 552. Capacitance module 552 is designed and constructed to have a capacitance that varies as a function of pressure applied to module 552. Capacitance measurement tool 510 is situated within container 510 an a manner which ensures that pressure applied to container 510, and causing deformation of the shape of container 510, applies pressure to capacitance module 552.

In the embodiment presented by Figure 10a, capacitance measurement tool 550 is embedded in container 510 in such a manner that distortions of the shape of container 510 have the effect of creating a lateral squeeze of capacitance module 552. The effect of being squeezed is to modify the capacitance of module 552, which change of capacitance is read and reported by capacitance measurement tool 550.

An alternative construction pictured in Figure 10b is similar to the construction presented in Figure 8b, in that a gas-filled partition 546 contains a capacitance module 552 implemented as pressure-sensitive capacitance module 553. Pressure on container 510 has the effect of compressing partition 546, raising the pressure of a gas contained therein, which change of pressure results in a change of capacitance of module 552, which is then read and reported by tool 550.

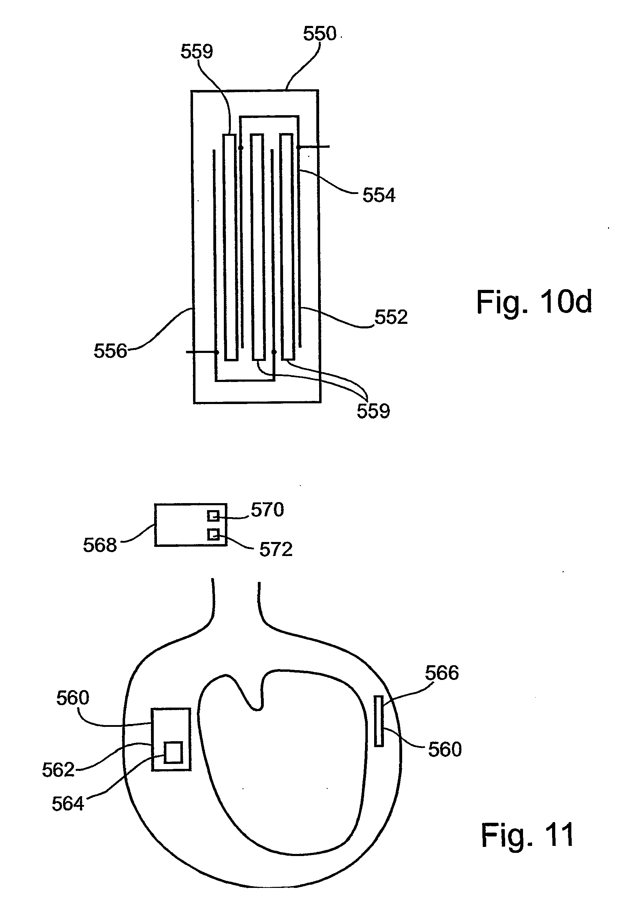

Figures 10c and 10d present, by way of example, alternative constructions for capacitance module 552. In the embodiment of Figure 10c, a first set of plates 554 is interleaved with a second set of plates 556. Plates 554 and 556 are able to move with respect to each other. Under lateral pressure such as is represented by arrows 558, plates 554 and plates 556 both slide towards the center of the device, thereby increasing an area of interleaving of plates 554 and 556, thereby increasing the capacity of module 552, which change of capacitance is read and reported by tool 550.

In the alternative construction presented in Figure 10d, a first set of plates 554 and a second set of plates 556 are separated by a spongy material 559. Spongy material 559 is compressible under pressure. Pressure applied to module 552 causes compression of material 559, thus bringing plates 554 into greater proximity with plates 556, thereby increasing capacitance of module 552, which change of capacitance is read and reported by tool 550.

Attention is now drawn to Figure 11, which is a simplified schematic of an additional preferred embodiment according to the present invention. In Figure 24, electronic measuring device 512 comprises an inductance module 560. Inductance module 560 comprises a tuned oscillator 562 including a coil 564, and further comprises a metallic element 566 electrically isolated from, but flexibly connected to, coil 254.

The flexible connection between metallic element 566 and coil 254 is such that changes in the shape of container 510 tend to cause modification of the spatial relationship between metallic element 566 and coil 254.

Modification of the spatial relationship between metallic element 566 and coil 254, and in particular modifications which cause metallic element 566 to approach nearer to coil 254 or alternatively to recede further from coil 254, cause, according to well-known laws of physics, changes in the inductance of coil 254.

Since coil 254 is part of tuned oscillator 562, modifying inductance of coil 254 modifies the tuning of tuned oscillator 562. Consequently, changes or deformations in the shape of container 510 under the influence of external forces such as pressure exerted on container 510 by muscle groups near a body cavity in which device 502 is inserted, produce changes in phase and frequency of oscillations of tuned oscillator 252.

Inductance module 560 further comprises a quantification module 568 operable to respond quantitatively to such a modifications of phase, or of frequency, of oscillations of tuned oscillator 252.

Quantification module 568 preferably comprises a reference oscillator 570 for providing a stable reference oscillation, which is used by a comparator 572 operable to compare an oscillation of reference oscillator 570 with an oscillation of tuned oscillator 562. Using comparator 572, quantification module 568 is operable to quantify differences in phase between oscillations of tuned oscillator 562 and oscillations of reference oscillator 570. Alternatively, using comparator 572, quantification module 568 is operable to quantify differences in frequency between oscillations of tuned oscillator 562 and oscillations of reference oscillator 570. Further alternatively, quantification module 568 may be operable to quantify both differences of phase and differences of frequency between oscillations of oscillators 562 and 570.

Quantification module 568 is presented in Figure 11 as being physically external to container 510, yet in alternate construction, some or all elements of quantification module 568 may be contained within container 510.

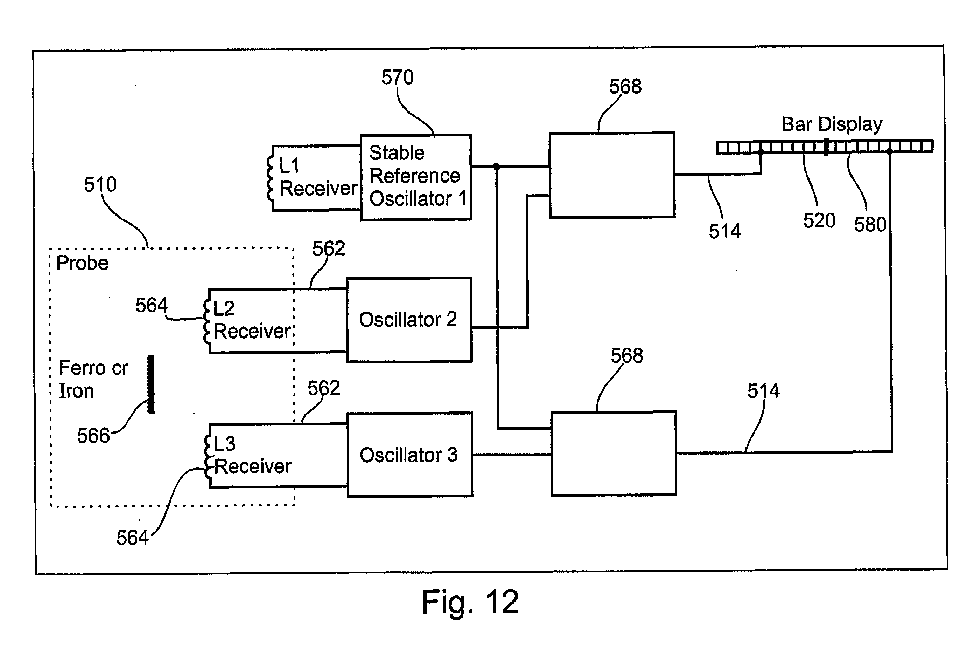

Reference is now made to Fig. 12 which is a simplified circuit diagram for use with the pressure sensing embodiment of the present invention. In Fig. 12 two oscillators 562 have variable frequencies depending on measured pressure of the device. The measured pressure may for example movement of iron element 566 relative to coils 564. A further, stable, oscillator 570 provides a reference signal. Comparators 568 are connected between each of the variable oscillators 562 respectively and the stable oscillator 570 to provide a comparative output indicative of variation in the oscillation rate. At its most simple, comparator 568 may be nothing more than an EXOR gate, the voltage or current of the EXOR output giving an indication of the pressure. The outputs of the comparators 568 are preferably connected to a bar display 580. The use of a bar display is particularly preferred because it provides results which are immediately apparent to the untrained eye. The bar display thus provides a form of very simple biofeedback in that the user is able to determine from the lights on the bar display whether she is succeeding in a given exercise.

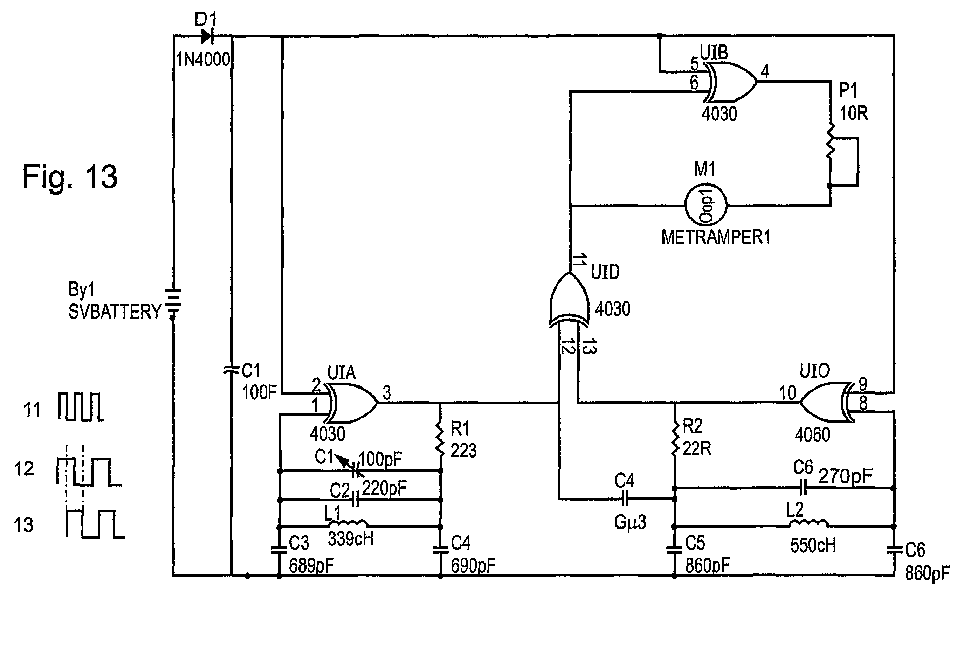

Attention is now drawn to Figure 13, which is a circuit diagram at component level showing in exemplary form how part of the diagram of Fig. 12 may be realized. Fig. 13 is simplified in that only a single variable oscillator is shown.

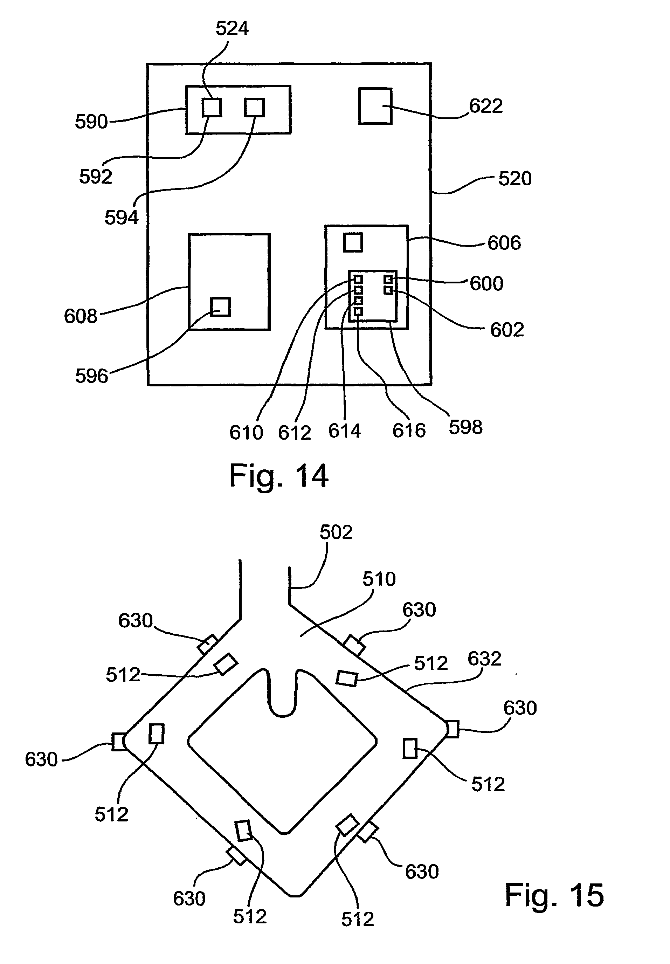

Attention is now drawn to Figure 14, which is a simplified block diagram presenting components of a data utilization system operable to utilize data provided by a distance measuring device, according to preferred embodiments of the present invention.

Figure 14 presents a data utilization system 520 operable to receive and utilize data generated by a measuring device 502.

In a preferred embodiment, data utilization system 520 comprises a display 590, typically comprising a visual display 592 and optionally additionally comprising an auditory display 594. Visual display 592 is typically useful to a user such as a medical practitioner utilizing device 502 for such purposes as medical diagnosis of a patient, or for monitoring progress of a medical treatment. Both visual display 592 and auditory display 594 are typically useful for providing feedback to a trainee monitoring his own body processes during a biofeedback training session. In a preferred embodiment, visual display 592 is a bar graph display 524.

In a preferred embodiment, data utilization system 520 further comprises a data recording facility 606 comprising mechanisms for storing data and for storing results of calculations. In a preferred embodiment, data utilization system 520 further comprises a calculation facility 608 for making calculations based on stored data or on data received from device 502.

Calculation facility 608 optionally comprises a microprocessor 596 operable for making calculations.

Data recording facility 606 comprises a memory 598 operable to store data. Stored data is typically utilized as a basis for calculations made by calculation facility 608, and may also be further used for delayed display.

Memory 598 preferably comprises a ram memory 600 for short-term storage and a hard disk 602 for long-term storage, and may optionally further comprise a flash memory 610, a bubble memory 612, a CD reader and recording device 614, a DVD reader and recording device 616, or any similar data storage device able to function as a data recording facility.

In a preferred embodiment, data recording facility 606 comprises a database 620.

In a preferred embodiment, data stored in database 620 during a first treatment of a patient may be compared by calculation facility 608 to data received from device 502 during subsequent treatment of a patient, the results of this comparison being displayed by display 590 in the form of a graphic comparison or in a form which emphasizes trends of development in the received data over time.

In a further preferred embodiment, data utilization system 520 comprises a biofeedback training system 622, operable to provide feedback to an operator regarding measurements of measured by device 502 consequent on voluntary actions by the user, thereby providing a reinforcement system for facilitating learning of voluntary control of selected body processes by the user.

Attention is now drawn to Figure 15, which is a simplified schematic showing additional preferred embodiments of a distance measuring system according to the present invention.

Figure 15 presents a distance measuring device 502, presented here as having a modified annular form characterized by a rounded diamond shape. In a preferred embodiment presented in Figure 15, device 502 comprises a plurality of electronic measurement devices 512. Distribution of a plurality of measurement devices 512 within device 502 presents the advantage that device 502, so configured, is enabled to simultaneously measure distances in a plurality of directions.

Device 502, in the various embodiments and configurations described hereinabove, has been primarily described as a tool for measuring distances. It is to be noted, however, that an important use for such a device is in the measurement of muscle strength and of muscle tone, for various muscles and muscle groups concentrating around various cavities of the human body. A typical use for device 502, for example, is in the diagnosis and treatment of urinary incontinence in women. It is well known that various types of urinary incontinence are typically associated with weakness and dysfunctionality of specific muscle groups. Device 502 can be used to quickly, easily, and consistently measure the strength of such muscle groups. For some diagnostic purposes, the distance of vaginal walls one from another, in various dimensions, can be considered to be correlated with muscular strength of those. For other purposes, measurements may be taken comparing distances in various directions measured without voluntary tensing of muscles, and compared to measurements in the same dimensions taken during tensing of muscles. In some diagnostic contexts, a large difference between the relaxed and the tensed state of the muscles can be taken as a measure of muscular strength. In other diagnostic contexts, a large difference between particular measures of distances between tensed and relaxed states may reflect a weakness in muscles which, had they been stronger, would have prevented such a large displacement.

A fruitful utilization of device 502 consists of measuring change in muscle strength as a response to therapeutic procedures. For one example, urinary incontinence is sometimes treatable by the use of Kegel exercises or other exercises. Yet it is widely known that Kegel exercises are difficult to learn to do properly, and that a large percentage of the patients attempting to treat urinary incontience through the use of such exercises do not reap the expected benefit because they do not properly execute the exercise. In this context, use of device 502 can benefit such patients both by providing immediate feedback during execution of an exercise as to the correctness of the exercise, and by providing long-term feedback to a patient by measuring changes in muscle strength over the course of a treatment program.

As described, urinary incontinence may in many cases be treated by means of electrical stimulation of muscles for the purpose of developing strength of the stimulated muscles and improved muscle tone throughout the urinary tract area. Device 502 may be useful in evaluating progress of such a treatment, by providing consistent measurements of muscle strength, comparable over time. System 500, comprising device 502 and a data utilization system as described hereinabove in the context of a discussion of Figure 14, is operative to show developmental trends in data recorded over a course of treatment.

Referring again to Figure 15, there is presented a configuration which is highly convenient when device 502 is used as a measurement device in the context of a course of therapeutic stimulation of muscles to treat urinary incontinence. Electrodes 630 of a therapeutic muscle stimulation system may be combined with the configurations of device 502 as described hereinabove, to provide a combine stimulation and measurement device 632, comprising both measurement configurations of device 502, and muscle stimulation configurations comprising electrodes for electrical stimulation of muscles, and other elements constituting an electrical muscle stimulation device.

Thus, the present embodiments provide *inter alia* a device utilizable for electrically stimulating the muscles and nerves defining and surrounding the intravaginal cavity. In sharp distinction to prior art devices, some of which are described in the background section hereinabove, the present invention present a simple solution for traversing the inherent limitations of prior art devices. By virtue of it's memory properties the device, according to the teachings of the invention: (i) self conforms to the anatomy of a wide range of individuals thus maintaining optimal contact between the electrode pairs provided thereupon and the wall of the intravaginal cavity; (ii) can be easily applied to, and removed from, the intravaginal cavity; and (iii) it remains at the desired location within the intravaginal cavity without producing discomfort while allowing the individual to be mobile without fear of device displacement.

The above embodiments thereby enable accurate stimulation of the muscles and nerves defining and surrounding the intravaginal cavity, and measurement of muscular activity, the measurement of muscular activity allowing biofeedback techniques as described above. It is particularly useful for treatment or prevention of urinary incontinence, a condition characterized by involuntary loss of urine; and/or (ii) imbalance of the autonomic nervous system versus the parasympathetic system, which is the cause of bladder dysfunction; both may benefit by such stimulation.

Thus electrical-stimulation and biofeedback monitoring provided by the device of the present invention can be utilized to effectively increase muscle strength while assessing muscle activity and prescribing the best course of treatment for the individual treated.

Additional objects, advantages, and novel features of the present invention will become apparent to one ordinarily skilled in the art upon examination of the following examples, which are not intended to be limiting. Additionally, each of the various embodiments and aspects of the present invention as delineated herein above and as claimed in the claims section below finds experimental support in the following examples.

### EXAMPLES

Reference is now made to the following examples, which together with the above descriptions illustrate the invention in a non-limiting fashion.

### Bladder Function

Bladder Function has two phases (Figures 22a-c); during the filling phase, the bladder stores urine until it can be emptied voluntarily, while during the emptying phase, the bladder releases its contents under voluntary control. Disorders that affect bladder storage (filling phase) cause urinary incontinence, whereas disorders of the emptying phase cause either partial or complete urinary retention. Disorders of both the filling and emptying phases are often found in the same patient, especially in older women.

Neurological control of bladder activity is extremely complex and is yet to be completely understood. The bladder and urethra are primarily innervated by the autonomic nervous system. Somatic nerves that innervate the pelvic floor muscles and the external urethral sphincter modulate this system. Control of continence relies upon the interplay between these two nervous systems and is coordinated by several centers in the brain and spinal cord.

### Filling Phase:

During the filling phase of bladder function, the detrusor muscle is at rest in a non-contractile state. The bladder has the ability to hold increasing volumes of urine (accommodation) without a concomitant rise in internal pressure. When accommodation is lost or impaired, spontaneous involuntary bladder contractions can result, leading to involuntary urine loss (Figures 21a-h). The spontaneous contractions, known as detrusor overactivity, unstable bladder, or detrusor instability, are a common cause of urinary incontinence.

The filling phase of bladder function is produced by sympathetic stimulation of β-adrenergic receptors within the bladder wall, which cause detrusor relaxation. At the same time, the sympathetic nervous system inhibits parasympathetic activity, further promoting a relaxed state. Detrusor relaxation during the filling phase is a key component for bladder accommodation. Sympathetic stimulation of the alpha-adrenergic receptors in the internal sphincter muscles causes constriction with a concomitant rise in urethral pressure.

The external urethral sphincter and levator ani muscles serve as backup continence mechanisms. Although in a constant state of contraction, these muscles can further contract during times of increased mechanical stress to forestall voiding for a short time. Both are influenced in a dual fashion from the pundendal nerve and sacral efferent nerves. Once the bladder reaches its functional capacity (usually 350 - 650 ml), receptors within the detrusor muscle signal higher cortical centers in the brain to initiate the emptying phase (or to continue the filling phase voluntarily).

### Emptying Phase:

Voiding, or micturition, depends on the coordinated activity of the urethra and detrusor muscle. Voiding is initiated by relaxation of the urethra and levator ani muscles, closely followed by a detrusor contraction. Inhibition of the pudendal and sacral efferent nerves produces relaxation of the external urethral sphincter and levator ani muscles Thus a posterior slackening of the supportive fascial hammock of the urethra eventuates. Meanwhile the cortex inhibits the sympathetic system and relaxation of the internal urethral sphincter occurs. The urethra shortens and that lowers resistance to urine flow. Activation of parasympathetic cholinergic receptors in the detrusor muscle stimulates bladder contraction, and micturition begins.

Emptying phase abnormalities may be due to a lack of timing between urethral relaxation and bladder contraction. Side effects of drugs or spinal cord disorders such as multiple sclerosis can result in urinary retention. Impaired contractility can occur with trauma, over-distention, fibrosis, or inflammatory processes that affect the bladder (Figures 21a-h). Lastly, voiding dysfunction can also result from increased outlet resistance caused by severe prolapsed organs trough the vagina or tumors.

The primary mechanism of continence (Figures 6a-8b), is produced by loops of specialized smooth muscle at the internal urethral sphincter. The urethra, resting on its supportive hammock of connective tissue (called the pubocervical fascia) is held in a position that prevents rotational descent (Urethrocele) into the vagina. Increases in intra-abdominal pressure compress the anterior urethral wall against the posterior urethral wall, which is fixed against the pubocervical fascial backstop. This action adds additional closing pressure to that generated by the internal sphincter mechanism. To further secure urinary continence, the external sphincter mechanism closes the middle portion of the urethra. The submucosal blood vessels plexus also plays a role in the prevention of leakage by sustaining a passive urethral pressure. Usually, incontinence only becomes clinically apparent when multiple failure of these systems take place.

### EXAMPLE 1

### Genuine Stress Incontinence

### Cause:

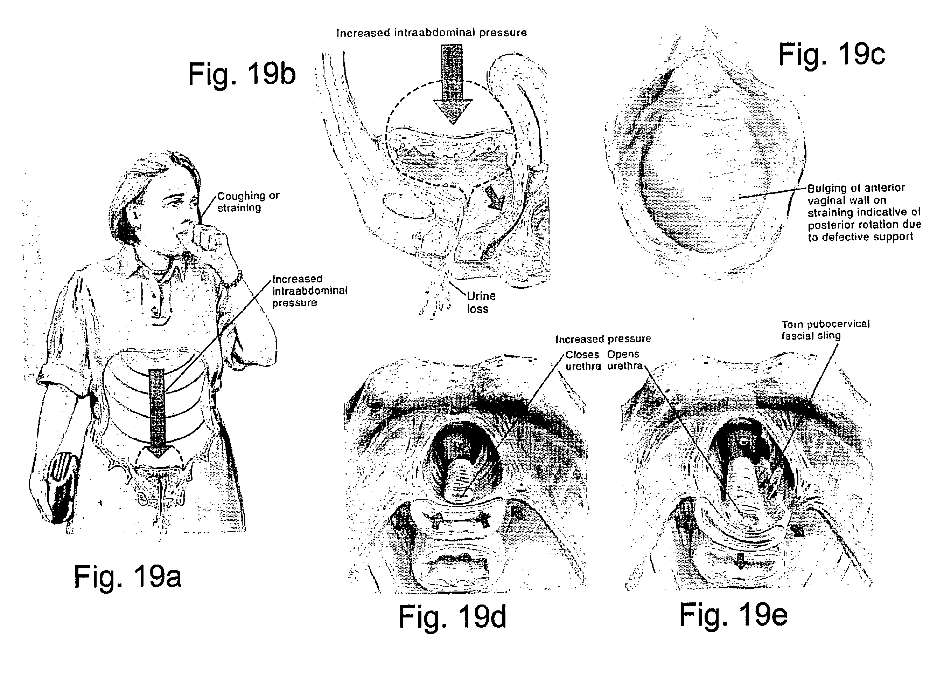

Involuntary loss of urine associated with increased intra-abdominal pressure (Figures 19a-e) is usually accompanied by a loss of support for the bladder neck/urethrovesical junction. On physical examination, this lack of support (urethrovesical junction hyper mobility, or as it is more often defined: bladder neck hyper-mobility) can be seen as a posterior rotational descent of the anterior vaginal wall into the vagina on straining.

A rare subtype of stress incontinence, termed intrinsic sphincter deficiency, is typified by paradoxical urethral sphincter opening during time of increased intra-abdominal pressure, even though the urethrovesical junction is relatively immobile on straining. Intrinsic sphincter deficiency is often seen in-patients who have had multiple anti-incontinence surgical procedures. Patients who have intrinsic sphincter deficiency often experience continuous leakage or leakage with minimal exertion.

### Treatment regimen:

According to the present invention, strengthening the pelvic floor muscles can heal mild and moderated genuine stress incontinence (unless the passive support of the connective tissue is too impaired and surgical repair is required). Positioning the device of the present invention within the vaginal cavity, such that each pair of electrodes is adapted to the different shape, location and unique direction of the muscle fibers is central for efficient ES treatment and SEMG reading. This ensures that the iliococcygeus and the Pubococcygeus (with the most important Puborectalis) muscles, which exhibit different location and direction, are treated separately by each pair of electrodes. Since the device of the present invention can be utilized to stimulate a weak muscle, whether unilaterally or bilaterally, it promotes the re-balancing of the hammock support of the pelvic organs, including the bladder neck. Furthermore, a repeated voluntary or artificial (via ES) contraction of the pelvic floor muscles has been found as an efficient strengthening factor to the existing supportive ligaments. Stimulation may also improve the re-innervation of partly denervated pelvic floor muscles by enhancing the regeneration of surviving motor axons.

The para and peri-urethral pelvic floor muscles contain slow, non-fatigable twitch muscle fibers (mostly in the external urethral sphincter, which is the extension of the Puborectalis muscle) which function is maintaining continence. The slow twitch muscle fibers have a fusion frequency of about 10-20 Hz. The para and peri-urethral pelvic floor muscles also contain fast-fatigable twitch muscle fibers (mostly the ilio and pubococcyegeus muscles), which produce 10 to 20 times more contraction force than the slow fibers. These muscles provide most of the closing force of the urethra during augmentation of intra-abdominal pressure (like coughing, sneezing etc.). Furthermore, the integrity of fast twitch muscle fibers is important for several other functions such as obtainment of normal orgasm, (which requires the ability of 0.8 contractions per second), and prevention of pelvic pain, such as vestibulitis, vulvodynia, urethral syndrome etc. The fast twitch muscle fibers have a fusion frequency of about 35-50 Hz.

It should be noted that when muscles are voluntary contracted, only 60 % of the fast-twitch-muscle-fibers are recruited. In contrast, ES treatment recruits all of the fast twitch muscle fibers, thus enabling more efficient training of such muscles fibers. Hence ES treatment achieves the desired strengthening goals within several weeks rather than the months it takes self exercise regimens.

An optimal incontinence treatment should include sequential or concomitant electrical stimulation of both fast and slow twitch muscle fibers. The device of the present invention enables such treatment since it allows the application of two unique and separate currents simultaneously or sequentially.

For example, Iliococcygeus stimulation via a 50Hz current provided from one or more pairs of electrodes, and puborectalis and/or external urethral sphincter stimulation via a 10 Hz current provided from a second pair of electrodes can be used as a suitable treatment regimen.

The stimulation and rest periods are selected according to need, or as detected by muscle feedback monitoring (e.g., SEMG - Surface Electro-Mayograph). If the muscles are very weak a 5 second stimulation/10 second rest interval is appropriate. If the muscles are strong and prevention is desired a 10second stimulation/10 second rest interval is used.

The duration of a treatment regimen is adapted according to individual needs. A short-term treatment regimen of 20 minutes/twice a day, with maximal intensity supportable, is optimal. A long-term treatment regimen, which is most suited for strengthening the musculature, would typically include an 8 hour/once a day treatment, at sub-sensational ES intensity. Although the latter treatment regimen is more efficient, it is less common, since prior art devices are typically uncomfortable and less accurate due to poor electrode positioning.

A typical duration of treatment is 12 weeks, and is recommended prior to elective surgical repair of defective connective tissue, since it has been previously shown that without the integrity of Puborectalis the recurrence of GSI occurs within 18 months in 50% of patients.

### EXAMPLE 2

### Detrusor Instability

### Cause:

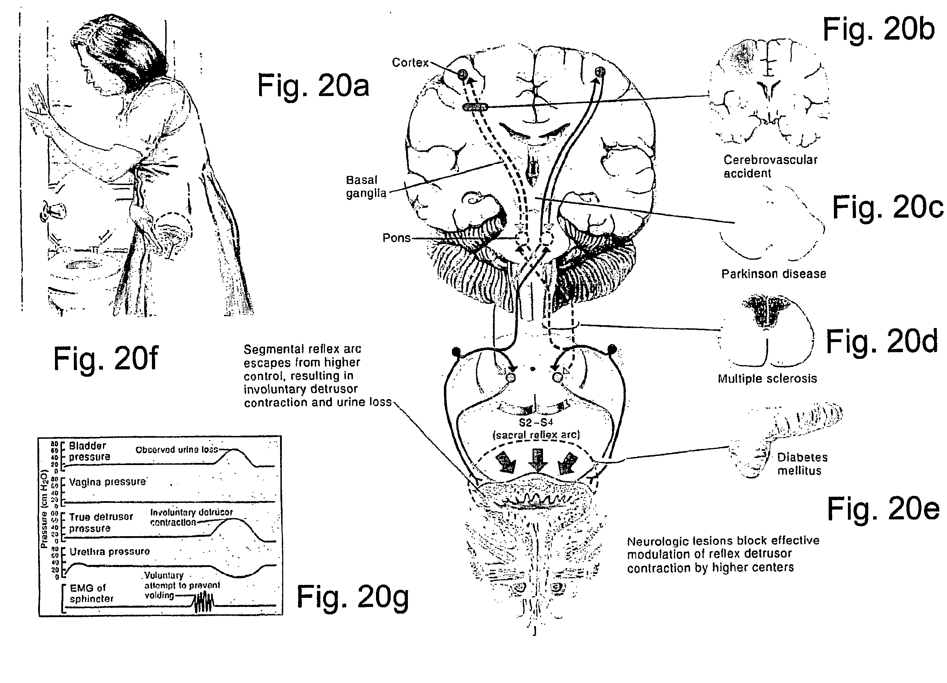

Incontinence with a strong urge to void stems from involuntary detrusor contractions, occurring during the filling phase (Figures 20a-g). Normally, either these contractions should not occur or the woman should be able to suppress the desire to urinate for a few minutes. "The bladder has escaped from the brain's control" - is a simple way to think about detrusor instability.

Since detrusor instability may result from several conditions, it has its own differential diagnosis. Symptoms suggesting bladder overactivity such as, frequency, urgency, and nocturia should be carefully assessed since they can sometimes herald potentially curable diseases.

For example, in older women, urinary tract infections (Figures 21g-h) can cause symptoms of urge incontinence, rather than the usual dysuria and suprapubic pain commonly seen in younger women. About 20% of women with bladder tumors (Figure 21g) present with the irritating symptoms of urge incontinence, rather than hematuria. Bladder stones (Figure 21g) can also produce similar symptoms and, unlike nephrolithiasis and ureterolithiasis, usually do not cause pain or hematuria. Bladder stones are most commonly found in nursing home residents who have urinary stasis and chronic bladder infections. Urge incontinence is also a result of suburethral diverticula (Figure 21g). These fluid-filled sacs are usually found along the posterolateral portion of the urethra. Diverticula can be multiple or single and can contain chronically infected urine, stones, or tumors. All the above pathologies present a contraindication to ES, but not to voluntary activation of the muscles, under biofeedback control. Lastly, prior anti-incontinence surgery, such as retropubic colposuspensions, can also result in urge incontinence (Figures 21b-e), as well as insufficient urethral closure pressure due to estrogen deficiency.

Although the diagnosis of detrusor instability typically implies that the patient is neurologicaly normal, some neurologic conditions can alter bladder function, resulting in unstable bladder contractions. Detrusor overactivity thought to be due to a neurologic disease is called detrusor hyperreflexia. Neurologic disorders that commonly cause detrusor instability are Alzheimer disease, parkinsonism, multiple sclerosis, and stroke.

### Treatment regimen:

The device of the present invention can be positioned within the vaginal cavity and used to treat (via ES) both the bladder sympathetic inhibitory system and the central inhibitory pathway to parasympathetic motor neuron. Both systems operate at rather low frequencies. Maximal inhibition via the sympathetic route can be obtained via a 5Hz current, whereas the central inhibitory pathway can be activated using a 5-10 Hz signal. The frequency characteristics are similar for reflexes elicited from the genital and anal region.

Phasic detrusor instability (or "idiopathic detrusor instability") which constitutes approximately 95% of all the detrusor hyper-activities, is a condition that results from minor imbalance between the bladder's positive-feedback system and the spinal inhibitory mechanism. Also, it is the most responsive of all the urinary pathologies to ES, without preferences to long-term ES treatment (8 hours a day of sub sensorial bi-phasic current), or maximal short-term ES treatment (20-30 minutes, at least twice a week).

The uninhibited overactive bladder, which is the non-voluntary loss of urine without any warning due to the lack of sensory projection between the pontine center and the frontal lobe (neurogenic dysfunction), is less amenable to treatment. As such, maximal stimulation is the therapy of choice.

The present invention proposes a new method for treating both sympathetic and central inhibitory pathways via ES stimulation. Simultaneous application of two unique frequencies (5 Hz and 10 Hz) to different tissue regions ensures maximal treatment efficiency.

### EXAMPLE 3

### Mixed Incontinence

### Cause:

Mixed Incontinence (GSI + DI) is the most common urinary incontinence disorder. Mixed incontinence is readily treatable via electric stimulation (ES) which induces multiple reflex effects restoring urinary control. An ES treatment regimen can be targeted to inhibit an overactive detrusor with concomitant improvement of urethral closure. A further advantage of ES treatment is that no outflow obstruction is created (unlike pessaries and some surgical procedures). Thus, ES seems to be the therapy of choice when combination of stress incontinence and Detrusor instability is present.

### Treatment regimen:

The present invention enables treatment via muscle awareness and voluntary training monitored by biofeedback (BFB); or muscle strengthening as well as bladder over-activity inhibition by ES. A unique feature of the present invention is its ability to treat both pathologies at the same time via a combined ES treatment regimen. An optimal treatment regimen in this case includes a 5-10 Hz signal for DI and a 50 Hz signal for GSI, for an interval including 5 seconds of stimulation and 10 seconds of rest.

The maximal short-term treatment regimen includes a 20-30 minute session/twice a day or at least twice a week. The long-term treatment regimen includes an 8hour/day stimulation, for 6 weeks to 12 weeks of treatment. Alternatively; a single current of an intermediate frequency of 20Hz can also be utilized.

It will be appreciated that such a treatment regimen is still recommended even in cases where surgical intervention is suggested, since following treatment according to the present invention, the patient becomes a more suitable candidate for surgery.

### EXAMPLE 4

### Overflow Incontinence

Involuntary loss of urine associated with an over-distended bladder can have a wide range of presentations, including frequent or constant urinary dribbling along with symptoms of stress and urge incontinence. Overflow incontinence can be due to outlet obstruction or may be secondary to an a-contractile or hypo-contractile detrusor muscle (Figure 21a-h).

Outlet obstruction is frequently encountered in men. It is rarely seen in women except when it is secondary to surgical procedures - retropubic colposuspensions (partial urethral obstruction), urethral procedures (urethral stenosis) - or severe pelvic prolapse (urethral kinking). When overflow incontinence does occur in women, it is more commonly associated with detrusor insufficiency or a-contractility, in which the bladder contraction is weak or nonexistent and voiding is incomplete or impossible. This condition can occur with certain drugs, fecal impacting, or after radical pelvic surgery. Neurologic diseases - such as diabetes, spinal cord tumors, and multiple sclerosis - can also cause overflow incontinence. Multiple sclerosis can have many effects on bladder health, causing urinary retention, incontinence from detrusor hyperreflexia, or both retention and leakage.

### Treatment regimen:

Overflow incontinence is not amenable to ES treatment, since it may increase bladder dysfunction by augmentation of hypo-contractility. However, the device of the present invention can be utilized to collect biofeedback in this case, to thereby increase awareness and ameliorate vesicourethral function.

### EXAMPLE 5

### Estrogen Deficiency

Estrogen status seems to play an important role in the continence mechanism and as such may be an important contributing factor to incontinence after menopause.

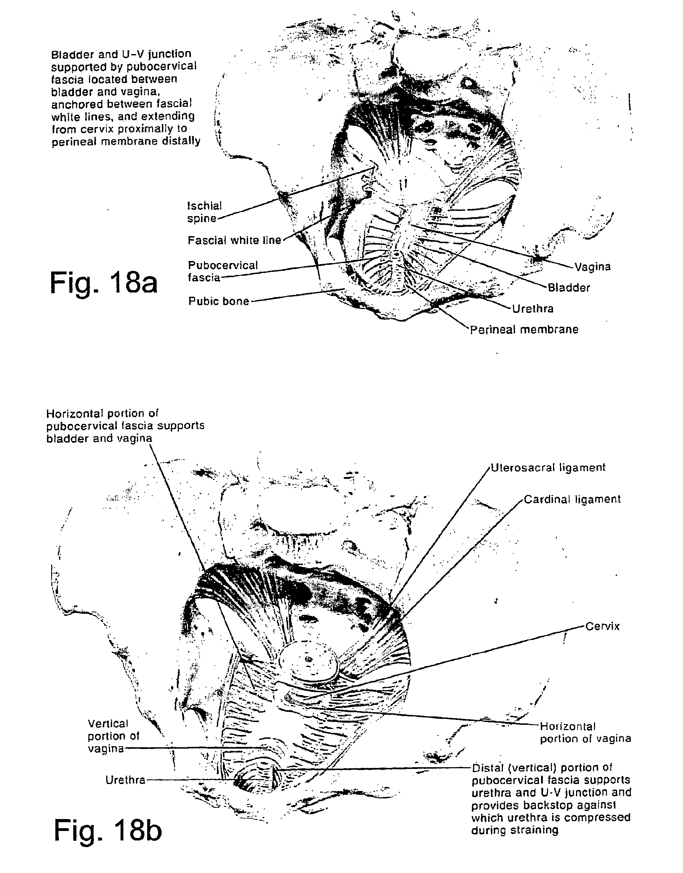

The lower urinary tract is rich in estrogen receptors. When these receptors are stimulated blood flow to the arteriovenous plexus, found in the submucosa of the urethra, is increased (Figures 18a-b). Augmented blood flow to this area enhances coaptation of the urethral mucosa and raises urethral pressure, promoting continence. Muscle activity is another factor that contributes to blood flow and thereby to urethral coaptation. It is well known that at the moment women stop benefiting from intercourse, due to any reason, the whole pelvic area begin to degrade and thus pathologies appear.

### Treatment regimen:

According to the present invention, incontinence caused by estrogen deficiency can be treated by pelvic floor activation, with any frequency and intensity that creates muscle fibers contraction, such that blood flow to the area will amplify the mucosal blood supply.

### EXAMPLE 6

### Vulvar diseases and pelvic pain

Pain in the pelvic region is very common. About 15% of the female population suffer from one or more forms of pelvic pain. Pelvic pain is diagnosed when such pain exists more than 6 weeks and does not respond to conventional medical treatment.

Pelvic pain may originate from the vulvar zone (vestibulitis, vulvodynia, dysparunia, episiotomy etc.), the vagina or the ano-rectal area (pudendal nevralgia, coccydinia, rectalgia etc.) and may be caused by infection, chronic regional pain, trauma and the like.

As well as being a source of great discomfort, pelvic pain oftentimes reduces a woman's will to have intercourse and as such, its short and long-term effects on conjugal life, self-image and quality of life can be significant.

### Treatment:

A common factor to all of these pathologies is the protective muscle spasm, which occurs as a defense reaction to pelvic pain. As a result, blood supply diminishes at the affected region and healing cannot take place. In addition to such changes in muscle neurological activity, the muscle becomes weak and unstable and it maintains higher tonus than normal.

Muscle training through BFB helps to normalize muscle tone, as does a suitable ES treatment which is targeted at creating a dermal block.

ES treatment can be used to eliminate pain thus breaking the vicious cycle of "pain-muscle spasm-pain". In addition, ES treatment helps the patient to regain normal neural activity of the pelvic floor muscles thus increasing their strength.

By applying a 200 ms pulse signal of 50-150 Hz, ES treatment can be utilized to effect segmental inhibition, which produces endorphin secretion through the peripheral effect of "Gate Control". The effect of such ES treatment is immediate but short lasting.

A central nervous system inhibitory effect can be obtained by ES stimulation of morphomimetic endogens secretion at the spinal cord fluid level. The ES parameters for such treatment include a 200-300 ms pulse signal of 1-10 Hz. Although such ES treatment does not produce immediate results, it produces sustained long-term effects.

Using the device of the present invention to provide an ES signal of 50Hz, for muscular regulation, concomitantly with an ES signal of about 10 Hz to alleviate pain and discomfort, an effective treatment can be provided.

### EXAMPLE 7

### Female Sexual Arousal Disorder

Female Sexual Arousal Disorder (FSAD) afflicts more than 20 million American women.

FSAD may be caused by psychological factors such as, low self esteem and/or physical factors such as inadequate blood flow to the erector bodies of the female sexual organ which may be caused by excessive smoking, or diabetes, or poor voluntary pelvic muscle fitness due to disuse atrophy, state post-traumatic history (such as vaginal birth, vaginal operations) or chronic muscle hypertension.

FSAD caused by poor voluntary pelvic muscle fitness typically results from an inability to prevent the escape of blood from the sensitized area during the arousal phase which results in inadequate blood flow to the erector bodies of the female sexual organ, or by failure to produce orgasm, which demand fast twitch muscle contraction of about 0.8 per second.

### Treatment:

Since blood flow in and around a tissue adjusts according to the demand of that tissue, stimulation of a specific group of muscles can be used to increase/modify blood supply to various tissues and to thereby treat FSAD.

To efficiently treat FSAD, control of the fast twitch muscle fibers must be acquired. This implies that the treatment regimen must be selected so as to enable rapid and complete recruitment of the muscle fibers, which recruitment depends upon anaerobic energy.

An ES treatment regimen including stimulation at 50Hz, recruits the fast twitch muscle fibers immediately, even in low volume, As such, stimulated muscles can be trained longer without fatigue. Thus, ES treatment using the device of the present invention achieves the desired strengthening and the necessary blood flow within few weeks instead of the months it takes commonly used exercise regimens.

In addition to providing the benefits of ES treatment, the novel configuration of the device of the present invention enables use anytime, anywhere while allowing the individual to be mobile without fear of device displacement.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims. In addition, citation or identification of any reference in this application shall not be construed as an admission that such reference is available as prior art to the present invention.

## Claims

1. A device (10) for interacting with muscles and nerves defining and surrounding an intravaginal cavity of an individual, the device comprising:
(a) a body (12) having memory properties such that when said body is contracted and positioned within an intravaginal cavity of the individual said body self expands in the intravaginal cavity; and
(b) at least one pair of electrodes (14) being attached to an exterior surface of said body (12) such that when said body is positioned within the intravaginal cavity, each electrode (14) of said at least one pair of electrodes is biased against a wall of the intravaginal cavity to thereby maintain electrical contact with said wall, wherein an electrical current providable from said at least one pair of electrodes serves for electrically stimulating the muscles and nerves defining and surrounding the intravaginal cavity, said at least one pair of electrodes including a plurality of electrode pairs;
said body (12) being constructed of an elastic material and **characterized in that** said body defines a substantially rounded diamond shaped frame when relaxed such that said body (12) conforms to a contour of the intravaginal cavity when expanding in the intravaginal cavity, each pair of electrodes (14) of said plurality of electrode pairs being attached to a specific region of said exterior surface of said body (12), such that each pair of electrodes is biased against a specific region of said wall of the intravaginal cavity when said body is positioned within the intravaginal cavity

2. The device of claim 1, placeable within the intravaginal cavity of an individual for treating a pelvic floor dysfunction in the individual.

3. The device of claim 1, further comprising a power and control unit (20) being for providing said electrical current to said at least one pair of electrodes (14), said power and control unit (20) being attachable to connectors (18) being in electrical communication with said at least one pair of electrodes (14).

4. The device of claim 3, wherein said connectors (18) are positioned on a portion of said body such that when said body (12) is positioned within the intravaginal cavity and attached to said power and control unit (20), said power and control unit is positioned outside of the intravaginal cavity.

5. The device of claim 1, wherein said body (12) is constructed of an elastic material selected from the group consisting of, rubber, latex and silicone.

6. The device of claim 1, wherein a width of said body (12) when relaxed is at least 1.3-1.5 times larger than said width of said body when fully contracted.

7. The device of claim 1, wherein a length of said body (12) when relaxed is 1.3-1.7 times shorter than said length of said body when fully contracted.

8. The device of claim 1, wherein said at least one pair of electrodes (14) includes an electrically conductive material selected from the group consisting of gold, carbon, stainless steel, silver and platinum.

9. The device of claim 3, wherein said power and control unit (20) further includes a controller (20) for controlling duration and/or intensity of electrical currents provided therefrom to said at least one pair of electrodes (14).

10. The device of claim 1, further comprising at least one sensor (24) attached to said body (12), such that when said body is positioned within the intravaginal cavity, said at least one sensor is capable of sensing muscle activity of the muscles defining and surrounding the intravaginal cavity.

11. The device of claim 10, wherein said at least one sensor (24) attached to said body (12) is a pressure sensor or an electrical activity sensor, or a displacement sensor.

12. The device of claim 10, wherein said at least one sensor (24) attached to said body further includes a transmitter.

13. The device of claim 12, wherein said transmitter serves for transmitting a signal receivable outside the body (12), said signal including data pertaining to said muscle activity sensed by said at least one sensor.

14. The device of claim 12, further comprising at least a second sensor (24).

15. The device of claim 11, further, comprising at least a second sensor (24).

16. The device of claim 11, said sensor (24) comprising an oscillator having a frequency variable in response to pressure applied thereto.

## Patentansprüche

1. Eine Vorrichtung (10) für das Interagieren mit Muskeln und Nerven, die eine intravaginale Kavität eines Individuums definieren und umgeben, wobei die Vorrichtung Folgendes umfasst:
(a) einen Körper (12), der Formgedächtnis-Eigenschaften aufweist, so dass, wenn der Körper kontrahiert und innerhalb einer intravaginalen Kavität des Individuums positioniert wird, der Körper in der intravaginalen Kavität selbst expandiert; und
(b) zumindest ein Paar an Elektroden (14), die mit einer äußeren Oberfläche des Körpers (12) verbunden sind, so dass, wenn der Körper innerhalb der intravaginalen Kavität positioniert wird, jede Elektrode (14) des zumindest einen Paars an Elektroden gegen eine Wand der intravaginalen Kavität vorgespannt ist, um **dadurch** elektrischen Kontakt mit der Wand aufrechtzuerhalten, wobei ein von dem einen Paar an Elektroden beziehbarer elektrischer Strom dem elektrischen Stimulieren der Muskeln und Nerven, welche die intravaginale Kavität definieren und umgeben, dient, wobei das zumindest eine Paar an Elektroden eine Vielzahl von Elektrodenpaaren einschließt;
wobei der Körper (12) aus einem elastischen Material konstruiert und **dadurch gekennzeichnet ist, dass** der Körper, wenn er entspannt ist, einen Rahmen mit im Wesentlichen der Form eines abgerundeten Diamanten definiert, so dass sich der Körper (12) einer Kontur der intravaginalen Kavität anpasst, wenn er in der intravaginalen Kavität expandiert, wobei jedes Paar an Elektroden (14) der Vielzahl von Elektrodenpaaren mit einem spezifischen Bereich der äußeren Oberfläche des Körpers (12) verbunden ist, so dass jedes Paar an Elektroden gegen einen spezifischen Bereich der Wand der intravaginalen Kavität vorgespannt ist, wenn der Körper innerhalb der intravaginalen Kavität positioniert wird.

2. Die Vorrichtung gemäß Anspruch 1, die innerhalb der intravaginalen Kavität eines Individuums für das Behandeln einer Funktionsstörung des Beckenbodens des Individuums platziert werden kann.

3. Die Vorrichtung gemäß Anspruch 1, die ferner eine Stromversorgungs- und eine Steuereinheit (20) umfasst, die dem Bereitstellen des elektrischen Stroms an das zumindest eine Paar an Elektroden (14) dient, wobei die Stromversorgungs- und Steuereinheit (20) mit Anschlüssen (18), die in elektrischer Verbindung mit dem zumindest einen Paar an Elektroden (14) stehen, verbunden werden können.

4. Die Vorrichtung gemäß Anspruch 3, wobei die Anschlüsse (18) auf einem Abschnitt des Körpers positioniert werden, so dass, wenn der Körper (12) innerhalb der intravaginalen Kavität positioniert wird und an die Stromversorgungs- und Steuereinheit (20) angeschlossen ist, die Stromversorgungs- und Steuereinheit außerhalb der intravaginalen Kavität positioniert wird.

5. Die Vorrichtung gemäß Anspruch 1, wobei der Körper (12) aus einem elastischen Material ausgewählt aus der Gruppe bestehend aus Gummi, Latex und Silikon konstruiert ist.

6. Die Vorrichtung gemäß Anspruch 1, wobei eine Breite des Körpers (12), in entspanntem Zustand, zumindest 1,3 bis 1,5 Mal größer als die Breite des Körpers, in vollständig kontrahiertem Zustand, ist.

7. Die Vorrichtung gemäß Anspruch 1, wobei eine Länge des Körpers (12), in entspannten Zustand, zumindest 1,3 bis 1,7 Mal größer als die Länge des Körpers, in vollständig kontrahiertem Zustand, ist.

8. Die Vorrichtung gemäß Anspruch 1, wobei das zumindest eine Paar an Elektroden (14) ein elektrisch leitfähiges Material ausgewählt aus der Gruppe bestehend aus Gold, Kohlenstoff, rostfreiem Stahl, Silber und Platin einschließt.

9. Die Vorrichtung gemäß Anspruch 3, wobei die Stromversorgungs- und Steuereinheit (20) ferner einen Controller (20) für das Steuern der Dauer und/oder Intensität von elektrischem Strom, der von derselben an das zumindest eine Paar an Elektroden (14) bereitgestellt wird, einschließt.

10. Die Vorrichtung gemäß Anspruch 1, die ferner zumindest einen Sensor (24), der mit dem Körper (12) verbunden ist, umfasst, so dass, wenn der Körper innerhalb der intravaginalen Kavität positioniert wird, der zumindest eine Sensor in der Lage ist, Muskelaktivität der Muskeln, welche die intravaginale Kavität definieren und umgeben, zu erfassen.

11. Die Vorrichtung gemäß Anspruch 10, wobei der zumindest eine Sensor (24), der mit dem Körper (12) verbunden ist, ein Sensor für Druck oder ein Sensor für elektrische Aktivität oder ein Wegsensor ist.

12. Die Vorrichtung gemäß Anspruch 10, wobei der zumindest eine Sensor (24), der mit dem Körper verbunden ist, ferner einen Sender einschließt.

13. Die Vorrichtung gemäß Anspruch 12, wobei der Sender dem Übertragen eines Signals, das außerhalb des Körpers (12) empfangen werden kann, dient, wobei das Signal Daten einschließt, die zu der von dem zumindest einen Sensor gemessenen Muskelaktivität gehören.

14. Die Vorrichtung gemäß Anspruch 12, die ferner zumindest einen zweiten Sensor (24) umfasst.

15. Die Vorrichtung gemäß Anspruch 11, die ferner zumindest einen zweiten Sensor (24) umfasst.

16. Die Vorrichtung gemäß Anspruch 11, wobei der Sensor (24) einen Oszillator mit einer Frequenz, die als Antwort auf darauf ausgeübten Druck variabel ist, umfasst.

## Revendications

1. Dispositif (10) pour interagir avec des muscles et des nerfs définissant et entourant une cavité intravaginale d'une personne, le dispositif comprenant :
(a) un corps 12 ayant des propriétés de mémoire telles que, lorsque ledit corps est contracté et positionné à l'intérieur d'une cavité intravaginale de la personne, ledit corps s'auto-dilate dans la cavité intravaginale ; et
(b) au moins une paire d'électrodes (14) qui sont fixées à une surface extérieure dudit corps (12) de telle sorte que, lorsque ledit corps est positionné à l'intérieur de la cavité intravaginale, chaque électrode (14) de ladite au moins une paire d'électrodes est sollicitée contre une paroi de la cavité intravaginale pour maintenir de cette façon un contact électrique avec ladite paroi, un courant électrique pouvant être fourni par ladite au moins une paire d'électrodes servant à stimuler électriquement les muscles et les nerfs définissant et entourant la cavité intravaginale, ladite au moins une paire d'électrodes comprenant une pluralité de paires d'électrodes ;
ledit corps (12) étant réalisé en une matière élastique et **caractérisé par le fait que** ledit corps définit un cadre en forme de losange sensiblement arrondi lorsqu'il est relaxé de telle sorte que ledit corps (12) épouse à un contour de la cavité intravaginale lorsqu'il se dilate dans la cavité intravaginale, chaque paire d'électrodes (14) de ladite pluralité de paires d'électrodes étant fixée à une région spécifique de ladite surface extérieure dudit corps (12), de telle sorte que chaque paire d'électrodes est sollicitée contre une région spécifique de ladite paroi de la cavité intravaginale lorsque ledit corps est positionné à l'intérieur de la cavité intravaginale.

2. Dispositif selon la revendication 1, apte à être placé à l'intérieur de la cavité intravaginale d'une personne pour traiter un dysfonctionnement du plancher pelvien dans la personne.

3. Dispositif selon la revendication 1, comprenant en outre une unité (20) de puissance et de commande qui est destinée à fournir ledit courant électrique à ladite au moins une paire d'électrodes (14), ladite unité (20) de puissance et de commande étant apte à être fixée à des connecteurs (18) qui sont en communication électrique avec ladite au moins une paire d'électrodes (14).

4. Dispositif selon la revendication 3, dans lequel lesdits connecteurs (18) sont positionnés sur une partie dudit corps de telle sorte que, lorsque ledit corps (12) est positionné à l'intérieur de la cavité intravaginale et fixé à ladite unité (20) de puissance et de commande, ladite unité de puissance et de commande est positionnée à l'extérieur de la cavité intravaginale.

5. Dispositif selon la revendication 1, dans lequel ledit corps (12) est réalisé en une matière élastique choisie dans le groupe constitué par le caoutchouc, le latex et le silicone.

6. Dispositif selon la revendication 1, dans lequel une largeur dudit corps (12) lorsqu'il est relaxé est au moins 1,3-1,5 fois plus grande que ladite largeur dudit corps lorsqu'il est totalement contracté.

7. Dispositif selon la revendication 1, dans lequel une longueur dudit corps (12) lorsqu'il est relaxé est 1,3-1,7 fois plus courte que ladite longueur dudit corps lorsqu'il est totalement contracté.

8. Dispositif selon la revendication 1, dans lequel ladite au moins une paire d'électrodes (14) comprend un matériau conducteur de l'électricité choisi dans le groupe constitué par l'or, le carbone, l'acide inoxydable, l'argent et le platine.

9. Dispositif selon la revendication 3, dans lequel ladite unité (20) de puissance et de commande comprend en outre un contrôleur (20) pour contrôler la durée et/ou l'intensité de courants électriques fournis à partir de celui-ci à ladite au moins une paire d'électrodes (14).

10. Dispositif selon la revendication 1, comprenant en outre au moins un détecteur (24) attaché audit corps (12), de telle sorte que, lorsque ledit corps est positionné à l'intérieur de la cavité intravaginale, ledit au moins un détecteur est capable de détecter une activité musculaire des muscles définissant et entourant la cavité intravaginale.

11. Dispositif selon la revendication 10, dans lequel ledit au moins un détecteur (24) fixé audit corps (12) est un détecteur de pression ou un détecteur d'activité électrique, ou un détecteur de déplacement.

12. Dispositif selon la revendication 10, dans lequel ledit au moins un détecteur (24) fixé audit corps comprend en outre un émetteur.

13. Dispositif selon la revendication 12, dans lequel ledit émetteur sert à émettre un signal apte à être reçu à l'extérieur du corps (12), ledit signal comprenant des données concernant ladite activité musculaire détectée par ledit au moins détecteur.

14. Dispositif selon la revendication 12, comprenant en outre au moins un second détecteur (24).

15. Dispositif selon la revendication 11, comprenant en outre un moins un second détecteur (24).

16. Dispositif selon la revendication 11, ledit détecteur (24) comprenant un oscillateur ayant une fréquence variable en réponse à une pression qui lui est appliquée.
